# EUROPEAN PATENT SPECIFICATION

(11) **EP 2 890 815 B1**
(45) Date of publication and mention of the grant of the patent: **20.03.2019**
(21) Application number: 13832208.6
(22) Date of filing: 30.08.2013
(51) Int. Cl.: C12Q 1/6886

(54) **METHODS FOR DIAGNOSIS AND TREATMENT OF CANCER**
VERFAHREN ZUR DIAGNOSE UND BEHANDLUNG VON KREBS
MÉTHODES DE DIAGNOSTIC ET DE TRAITEMENT DU CANCER

(30) Priority: 31.08.2012 US 201261696002 P; 24.05.2013 US 201361827514 P
(43) Date of publication of application: 08.07.2015
(73) Proprietor: The Regents of the University of Colorado, Denver, CO 80203 (US)
(72) Inventor: DOEBELE, Robert, C., Denver, CO 80209 (US); GARCIA, Marileila, Varella, Greenwood Village, CO 80111 (US); LE, Anh, T., Denver, CO 80206 (US)
(74) Representative: Steinecke, Peter
(86) International application number: PCT/US2013/057495
(87) International publication number: WO 2014/036387

(56) References cited:
- WO-A2-2014/071358
- US-A1- 2004 180 811
- US-A1- 2011 118 298
- US-A1- 2011 136 683
- US-A1- 2012 208 713
- US-B2- 8 232 060
- MARTA BUTTI ET AL: "A sequence analysis of the genomic regions involved in the rearrangements between TPM3 and NTRK1 genes producing TRK oncogenes in papillary thyroid carcinomas", GENOMICS, vol. 28, 1 July 1995 (1995-07-01), pages 15-24, XP055261865,
- A. GRECO ET AL: "Rearrangements of NTRK1 gene in papillary thyroid carcinoma", MOLECULAR AND CELLULAR ENDOCRINOLOGY, vol. 321, no. 1, 1 May 2010 (2010-05-01), pages 44-49, XP055176824, ISSN: 0303-7207, DOI: 10.1016/j.mce.2009.10.009
- LISA HUTCHINSON: "Lung cancer: Drug-sensitivity-time for a rearrangement?", NATURE REVIEWS CLINICAL ONCOLOGY, vol. 10, no. 12, 12 November 2013 (2013-11-12), pages 670-670, XP055261015, NY, US ISSN: 1759-4774, DOI: 10.1038/nrclinonc.2013.216
- ARIA VAISHNAVI ET AL: "Oncogenic and drug-sensitive NTRK1 rearrangements in lung cancer", NATURE MEDICINE, vol. 19, no. 11, 27 October 2013 (2013-10-27), pages 1469-1472, XP055102091, ISSN: 1078-8956, DOI: 10.1038/nm.3352
- MANUEL R. TEIXEIRA: "Recurrent Fusion Oncogenes in Carcinomas", CRITICAL REVIEWS(TM) IN ONCOGENESIS, vol. 12, no. 3-4, 1 January 2006 (2006-01-01), pages 257-271, XP055105339, ISSN: 0893-9675, DOI: 10.1615/CritRevOncog.v12.i3-4.40
- PEIFER, M. ET AL.: 'Integrative Genome Analyses Identify Key Somatic Driver Mutations Of Small-Cell Lung Cancer.' NATURE GENETICS vol. 44, 02 September 2012, pages 1104 - 1110, XP002699655

## Description

### FIELD OF THE INVENTION

The present invention generally relates to markers, methods and assay kits for the identification of lung cancer patients predicted to respond to specific cancer therapies.

### BACKGROUND OF THE INVENTION

Lung cancer remains a leading cause of mortality in cancer worldwide and is mostly represented by non-small cell lung cancer (NSCLC). NSCLC is increasingly being recognized as a heterogeneous set of diseases based both upon histology as well as molecular characteristics. The identification of these molecular subsets is relevant as there is a growing number of targeted therapies that can effectively inhibit activated oncogenes leading to improved clinical outcomes for patients.

The first important oncogenic fusion in lung cancer was discovered in 2007 by Soda and colleagues. The anaplastic lymphoma kinase gene (ALK) was activated by fusion with the echinoderm microtubule-associated protein-like 4 (EML4). This fusion gene resulted in constitutive activation of the ALK tyrosine kinase domain with activation of downstream signaling pathways and transformed cell growth. Since this discovery, TFG and KIF5B have also been identified as fusion partners for ALK in NSCLC. Crizotinib, a tyrosine kinase inhibitor (TKI) with multiple targets, is currently approved by the US FDA for the treatment of patients with advanced NSCLC proven to be ALK positive (ALK+) and the only FDA approved method for identifying ALK positivity is fluorescence in situ hybridization (FISH) using break-apart (BA) probes specific for regions 5' and 3' of the common breakpoint in rearranged ALK. ROS1 is another receptor tyrosine kinase (RTK) recently found to be activated by gene fusions in NSCLC. The first cancer-related genomic rearrangement involving ROS1, an intra-chromosomal deletion on chromosome 6q21 fusing the 5' region of GOPC to the 3' region of ROS1, was reported in glioblastoma. In the last five years, 7 different fusions activating ROS1 were identified. Importantly, the ROS1 kinase domain is retained in all of these fusion events and the expressed fusion genes have been reported to be oncogenic. FISH has been a technical platform commonly used to diagnosis these rearrangements, as the nature of the assay allows it to, in theory, detect any all cases in which the ROS1 gene has undergone rearrangement. Recent data also support that lung cancer patients harboring ROS1 gene fusions also respond to crizotinib supporting the clinical utility in identifying these patients.

Four recent studies describe the 3rd gene activated by fusion in lung cancer, RET (Rearranged during Transfection). RET is a well known RTK, with an oncogenic role in papillary and follicular thyroid carcinoma through activation by gene fusions (RET-PTC). Patients whose tumors harbor this fusion respond well to vandetanib, an oral TKI that targets VEGFR, EGFR and RET. A total of 33 patients were reported harboring KIF5B-RET fusions involving 7 different breakpoints. These molecular rearrangements were identified through multiple technologies used to screen more than 2,000 lung adenocarcinomas. In the 3 larger series screened, likely with lower level of pre-selection, frequency of KIF5B-RET fusion ranged from 1% to 2%. A further study describes a gene fusion in thyroid carcinoma in which NTRK1 is involved (Butti M. et al.GENOMICS, vol. 28, 1 July 1995, p. 15-24).

There continues to be a need in the field for identification of further molecular markers, including oncogenic fusion markers, to facilitate more effective detection and treatment of lung cancer.

### SUMMARY OF THE DISCLOSURE

The present invention is based on the discovery of a novel gene fusion comprising the *NTRK1* gene that is indicative of lung cancer and also indicative of which patients may respond to cancer therapy comprising therapeutic administration of tyrosine kinase inhibitors.

Accordingly, in one embodiment, the invention comprises a method for determining if a lung cancer patient is predicted to respond to the administration of a chemotherapeutic regimen. The method comprises detecting in a sample of tumor cells from the patient the presence or absence of a marker, wherein the marker comprises a gene fusion comprising a *NTRK1-MPRIP* gene fusion (NTRK1 encodes the TRKA protein), and wherein the presence or absence of the marker is indicative of whether the cancer patient will respond to the administration of the chemotherapeutic regimen.

In various embodiments, the chemotherapeutic regimen may include administration of one or more of the following: a tyrosine kinase inhibitor, a HSP90 inhibitor (or other chaperone inhibitor), an inhibitor that targets tyrosine kinase downstream signalling cascade, or combinations thereof. Such inhibitors are well known in the art and are commercially available. All such inhibitors are encompassed in the present invention. For instance, in some embodiments, the tyrosine kinase inhibitor may be a TrkA inhibitor, examples of which include, but are not limited to, crizotinib (PF-02340166), ponatinib (AP24534), dovitinib (TK-258), CEP-701, or rebastinib (DCC-2036). Examples of HSP90 inhibitors include, but are not limited to, geldanamycin, herbimycin, 17-AAG, PU24FCl, STA-9090, IPI-504, and AUY-922. Examples of inhibitors that target tyrosine kinase receptor downstream signalling cascade include, without limitation, elumetinib (AZD6244) and MK2206.

In some embodiments, a level of the marker is determined and compared to a standard level or reference range. In some embodiments, the standard level or reference range is determined according to a statistical procedure for risk prediction.

In some embodiments, the presence of the marker may be determined by detecting the presence of a polynucleotide or a polypeptide. In some embodiments, the method may comprise detecting the presence of the polypeptide using a reagent that specifically binds to the polypeptide or a fragment thereof. The reagent may be an antibody, an antibody derivative, or an antibody fragment.

In some embodiments, the presence of the marker may be determined by obtaining RNA from the sample; generating cDNA from the RNA; amplifying the cDNA with primers specific for the marker; and determining from the sequence of the amplified cDNA the presence of absence of the marker in the sample. In some embodiments, the presence of the marker may be determined by Fluorescent In Situ Hybridization (FISH).

The methods of the present invention may further comprise comparing the expression level of the marker in the sample to a control level of the marker selected from the group consisting of: a) a control level of the marker that has been correlated with beneficial response to the administration of a chemotherapeutic regimen including one or more kinase inhibitor(s); and a control level of the marker that has been correlated with lack of beneficial response to the administration of a chemotherapeutic regimen including one or more kinase inhibitor(s); and b)selecting the patient as being predicted to respond to the administration of a chemotherapeutic regimen including one or more kinase inhibitor(s), if the expression level of the marker in the sample is statistically similar to, or greater than, the control level of expression of the marker that has been correlated with sensitivity to the administration of a chemotherapeutic regimen including one or more kinase inhibitor(s), or c) selecting the patient as being predicted to not respond to the administration of a chemotherapeutic regimen including one or more kinase inhibitor(s), if the level of the marker in the sample is statistically less than the control level of the marker that has been correlated with beneficial response to the administration of a chemotherapeutic regimen including one or more kinase inhibitor(s).

In some embodiments, the methods may further comprise comparing the expression level of the marker in the sample to a level of the marker in a second patient predicted to not respond to the administration of a chemotherapeutic regimen including one or more kinase inhibitor(s), and, selecting the patient as being predicted to respond to the administration of a chemotherapeutic regimen including one or more kinase inhibitor(s), if the expression level of the marker in the sample is greater than the level of expression of the marker in the second patient, or, selecting the patient as being predicted to not respond to the administration of a chemotherapeutic regimen including one or more kinase inhibitor(s), if the level of the marker in the sample is less than or equal to the level of expression of the marker in the second patient. In some embodiments the patient is human.

In a further aspect, the present disclosure includes an assay system for predicting patient response or outcome to tyrosine kinase anti-cancer therapy comprising a means to detect at least one of: a) the presence of a gene fusion comprising a *NTRK1-MPRIP* gene fusion; b) the level of expression of a gene transcript encoded by a *NTRK1-MPRIP* gene fusion; c) the presence of a protein encoded by a *NTRK1-MPRIP* gene fusion; d) the level of a protein encoded by a *NTRK1-MPRIP* gene fusion; and, e) the activity of a protein encoded by a *NTRK1-MPRIP* gene fusion. In some embodiments, the means to detect comprises nucleic acid probes comprising at least 10 to 50 contiguous nucleic acids of *NTRK1* gene, or complementary nucleic acid sequences thereof. In some aspects, the means to detect comprises binding ligands that specifically detect polypeptides encoded by a *NTRK1-MPRIP* gene fusion. In some aspects, a surface of the assay system comprises a chip, array, or fluidity card. In some aspects, the assay system further comprises: a control selected from the group consisting of: information containing a predetermined control level of a gene transcript encoded by a *NTRK1-MPRIP* gene fusion that has been correlated with response to the administration of a chemotherapeutic regimen including one or more kinase inhibitor(s); and information containing a predetermined control level of a gene transcript encoded by a *NTRK1-MPRIP* gene fusion that has been correlated with a lack of response to the administration of a chemotherapeutic regimen including one or more kinase inhibitor(s).

In another embodiment, the present invention includes a method of diagnosing a specific type of lung cancer in a subject, comprising detecting in a sample of cells from the subject the presence of a *NTRK1-MPRIP* gene fusion marker, wherein the presence of the marker is indicative of whether the subject has the specific type of lung cancer. In some embodiments, the presence of the gene fusion marker is detected by RT-PCR or FISH. In some embodiments, the presence of the gene fusion marker is detected by detecting the polypeptide encoded by the gene fusion marker. In some embodiments, the polypeptide is detected by using a reagent that specifically binds to the polypeptide or a fragment thereof.

Other features and advantages of the invention will become apparent to one of skill in the art from the following detailed description.

### BRIEF DESCRIPTION OF THE DRAWINGS

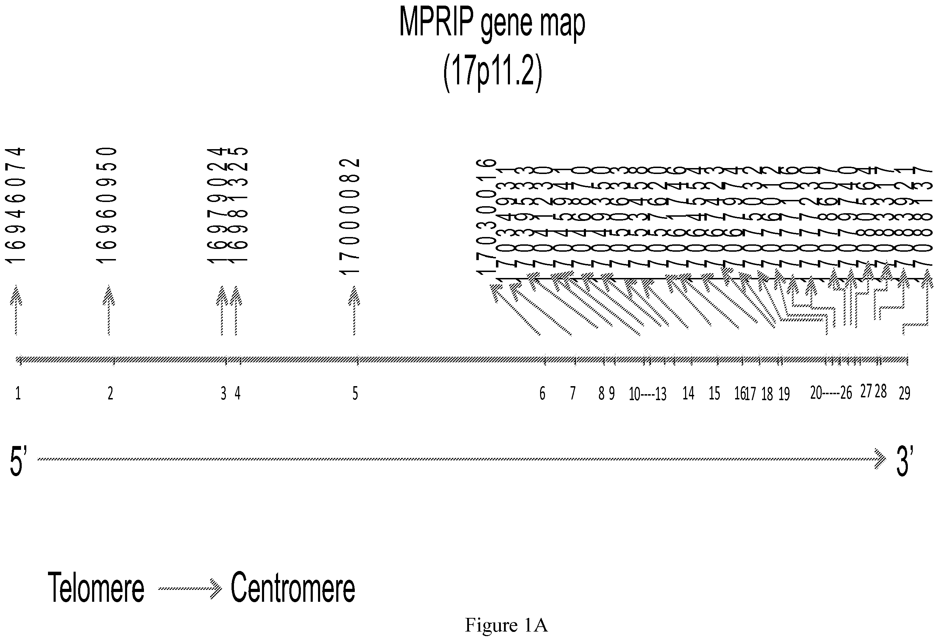

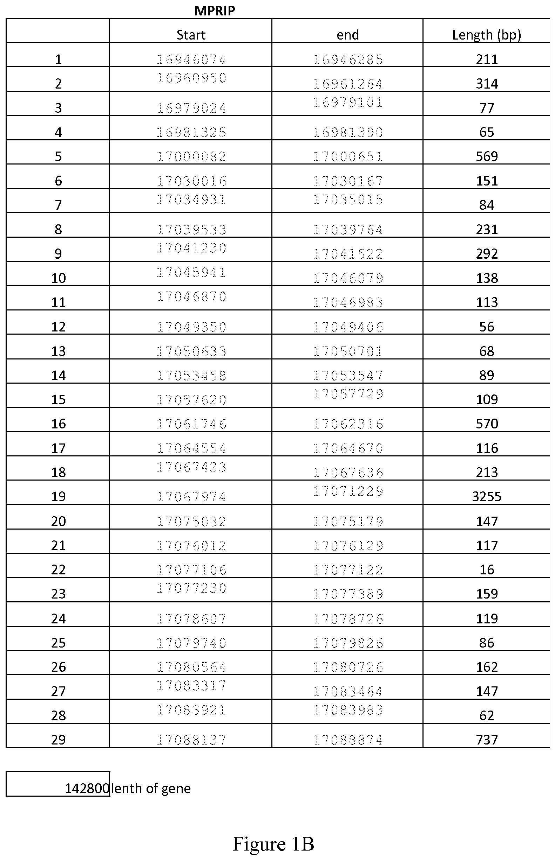

Figure 1 shows the chromosomal and exon maps of *MPRIP* gene.

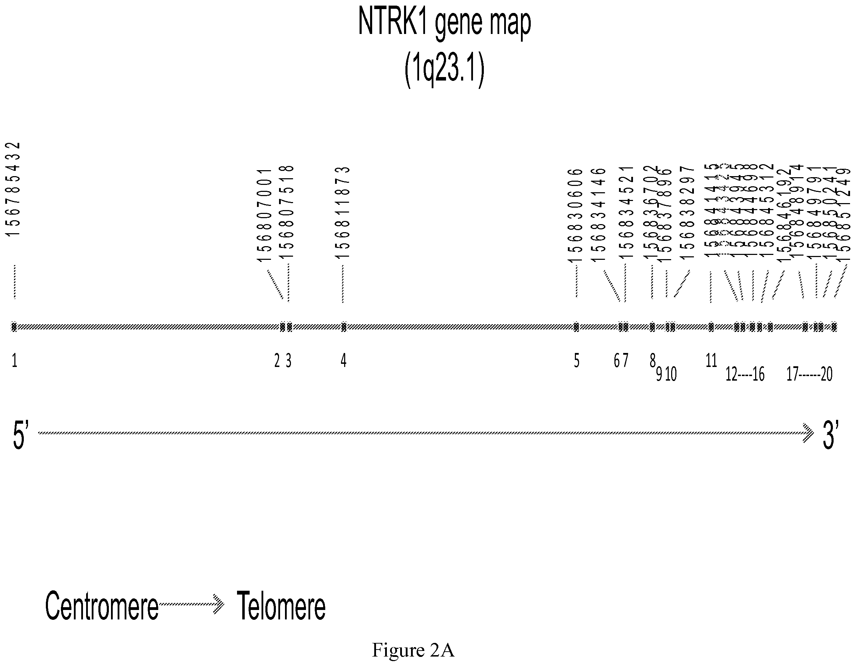

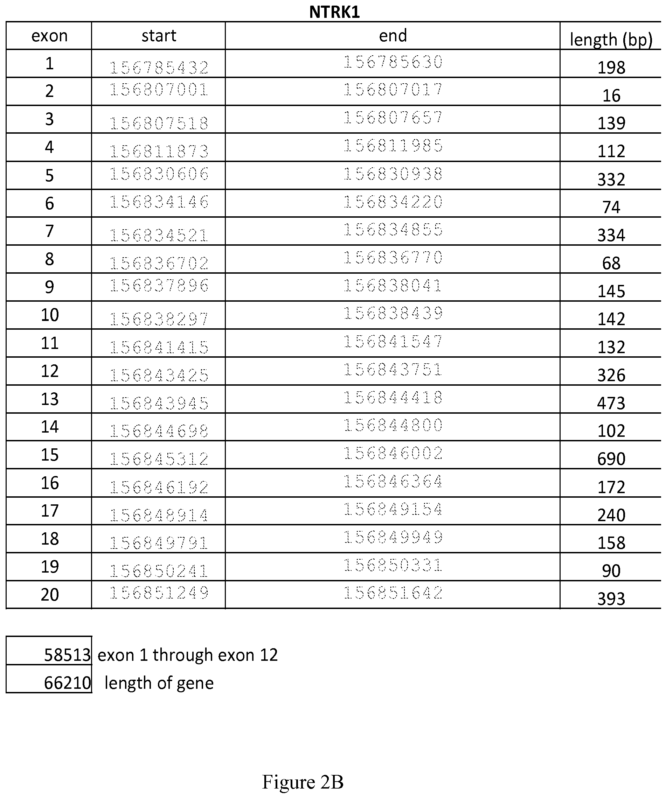

Figure 2 shows the chromosomal and exon maps of *NTRK1* gene.

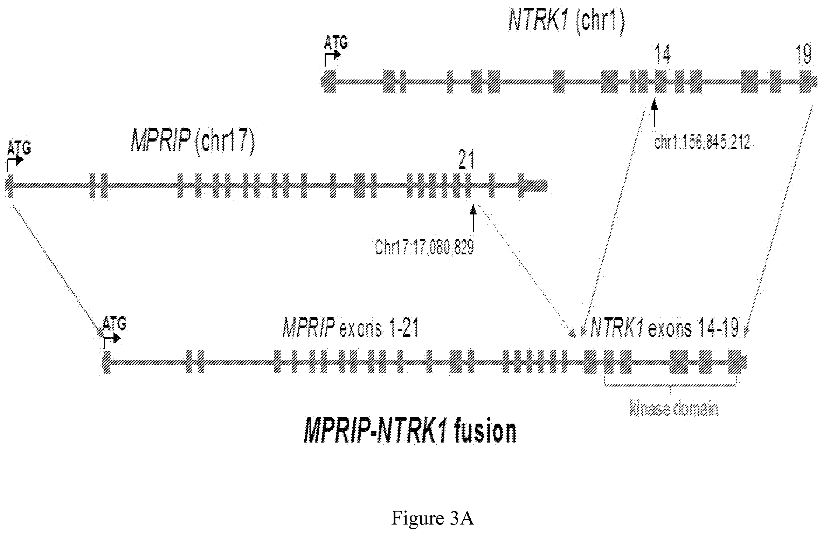

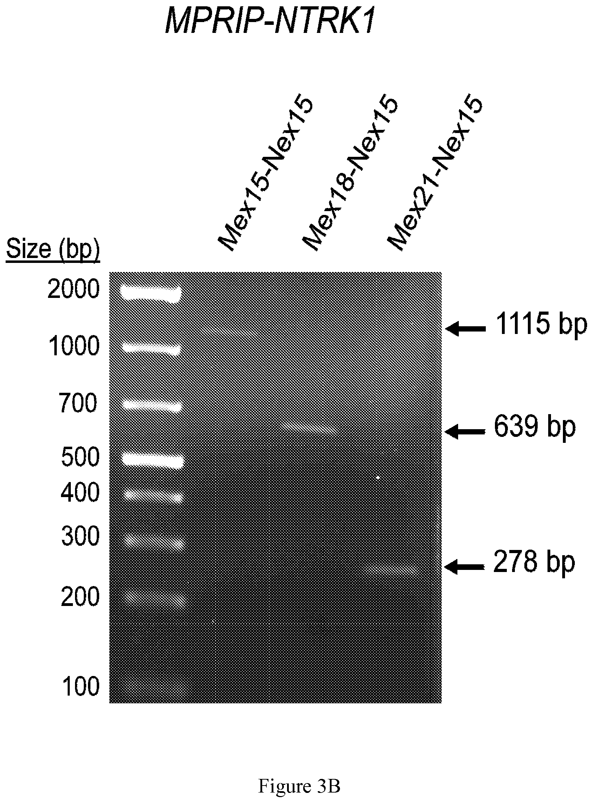

Figure 3 shows the arrangement and data confirming NTRK1 gene fusions in lung cancer samples. Figure 3A is a schematic of genomic rearrangement from tumor samples harboring NTRK1-MPRIP. Figure 3B shows RT-PCR demonstrating mRNA expression of the novel fusion transcripts. RNA extracted from formalin-fixed paraffin-embedded tumor sample harboring the NTRK1-MPRIP was subject to RT-PCR followed by agarose gel electrophoresis and DNA sequencing. The following abbreviates are used: MPRIP (M), CD74 (C), NTRK1 (N), and exon (ex). Sanger sequencing chromatograms were obtained of RT-PCR product of RNA isolated from tumor samples with NTRK1-MPRIP fusion. SEQ ID NO:1 is the complete cDNA sequence of NTRK1-MPRIP fusion (M21;N14). The cDNA was cloned from a frozen tumor sample from the patient in which this fusion was first identified. Capital letters represent nucleotides contained within the open reading frame.

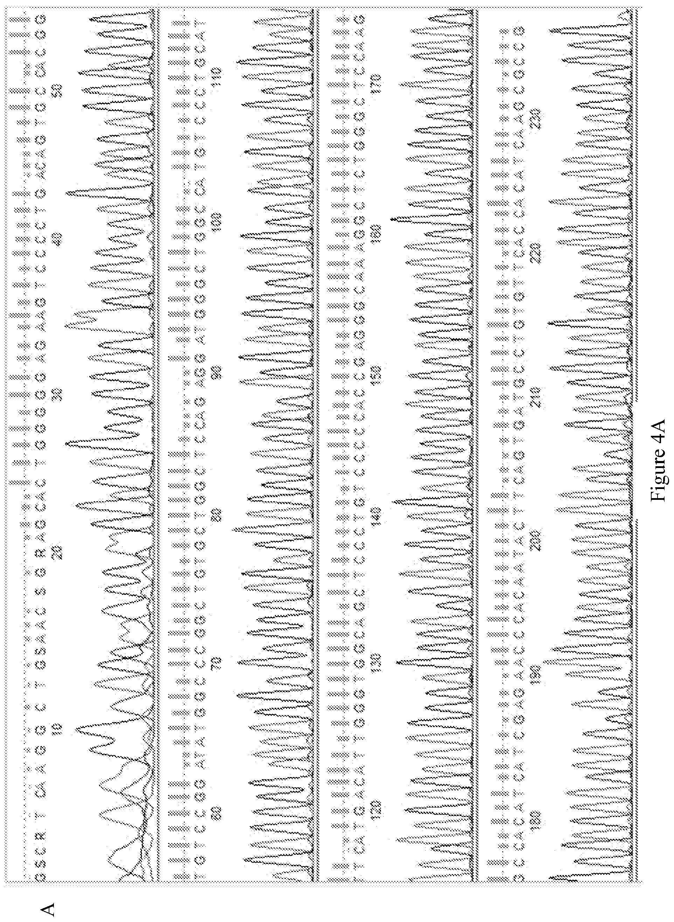

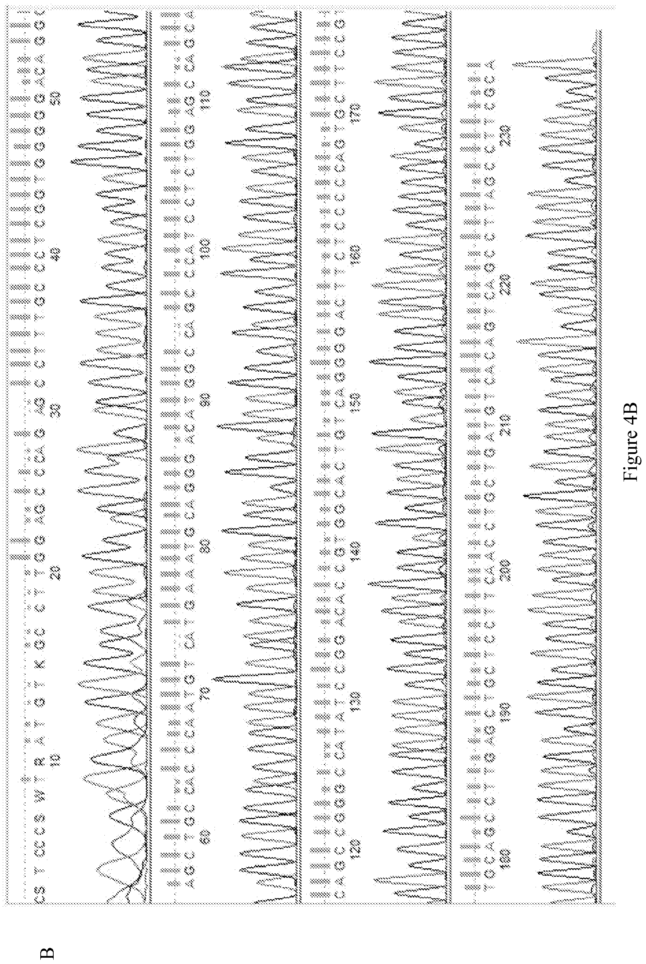

Figure 4 shows the RT-PCR primer read sequences for detection of *NTRK1-MPRIP* gene fusion. Figure 4A shows the forward read sequence with *MPRIP* CC3F1 primer. Figure 4B shows the reverse read sequence with *NTRK1* Y490R1 primer.
Figure 5 shows the design of the *NTRK1-MPRIP* fusion FISH probes.
Figure 6 shows the design of the 5'-3' *NTRK1* Break-Apart FISH probe set aligned against the NTRK1 encoding region of chromosome 1q23.1.
Figures 7A and 7B show FISH images obtained from the normal cell line GM09948 using the *NTRK1-MPRIP* gene fusion probes, providing single clone validation in a normal cell line ( Diploid,2N). Figure 7A: Clone RP11-1038N13 (3'NTRK1) and RP11-125116 (5'MPRIP). Figure 7B: Clone RP11-1059C21 (3'NTRK1) and Rp11-796J19 (5'MPRIP).
Figures 8A and 8B show FISH images obtained from tissue sections that are negative for *NTRK1-MPRIP* gene fusion using the *NTRK1-MPRIP* gene fusion probes. Figure 8A shows specimen S-12-047486 showing low copy number for both genes. Figure 8B shows specimen S-12-047098, showing high copy number for 3' NTRK1 and Mid/low copy number for 5' MPRIP.
Figures 9A and 9B show FISH images obtained from tissue sections that are positive for *NTRK1-MPRIP* gene fusion using the *NTRK1-MPRIP* gene fusion probes.

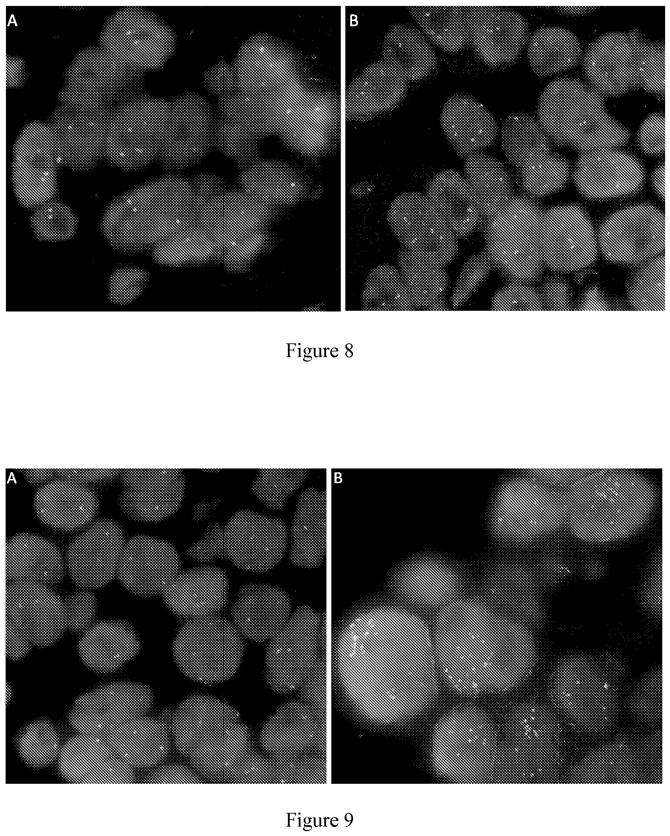

Figure 9A showing low copy number for both genes with approximately one red/green fusion (positive pattern) per tumor cell. Figure 9B showing gene amplification of the fused red/green with mid/low copy number of single reds and single greens per tu or cell.

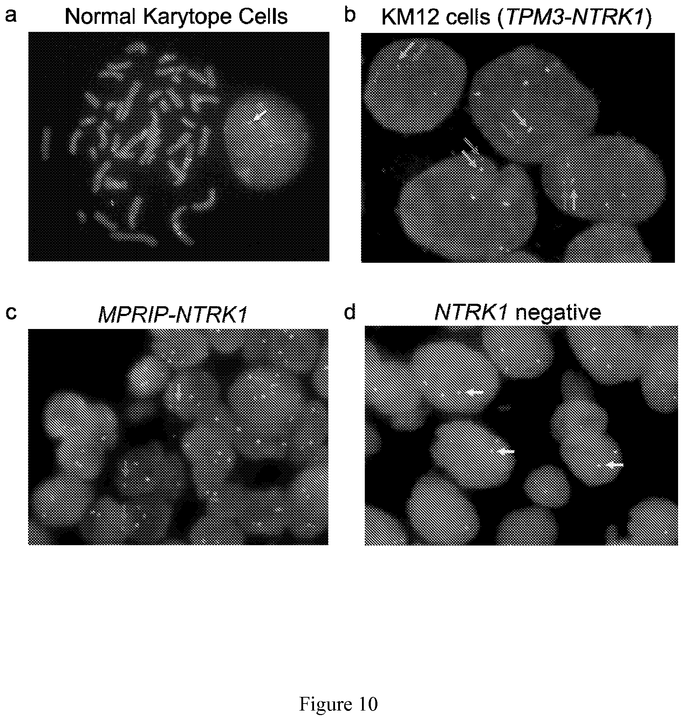

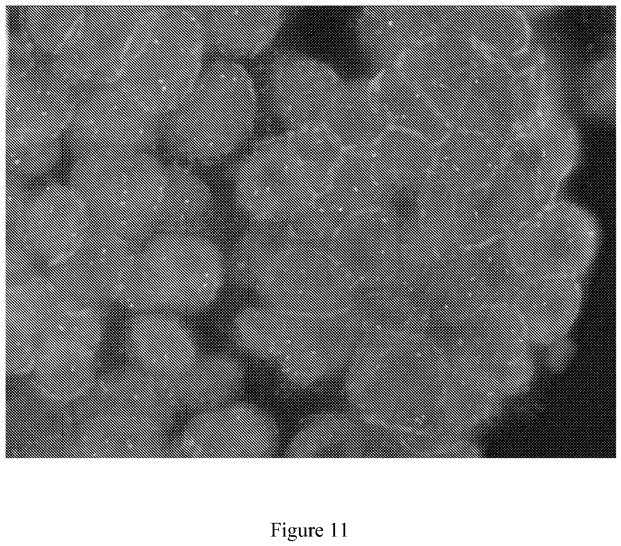

Figure 10 shows the testing of NTRK1 break-apart FISH probe. Figure 11a shows cell line GM09948 with a normal karyotype showing metaphase spread and interphase nuclei demonstrating close proximity of the 5' and 3' signals indicating an intact NTRK1 gene. Figure 11b shows KM12 cells which harbor a TPM3-NTRK1 gene fusion showing clear separation of the 5' and 3' signals indicating a rearrangement of the NTRK1 gene. Figure 11c shows a break-apart FISH analysis of NTRK1-MPRIP samples showing clear separation of 5' and 3' signals corresponding to the NTRK1 gene. Figure 11d shows break-apart FISH analysis of a tumor sample without an NTRK1 gene rearrangement showing close approximation of the green/red signals (indicated by arrow).
Figure 11 shows FISH images obtained from tissue sections that are positive for *NTRK1* gene rearrangement using the *NTRK1* break-apart probes. Specimen S12-6889 B1 Hybridized with the 5'NTRK1/3'NTRK1 Break Apart probe set. Cells show both the 'positive' pattern of split and the 'normal' pattern of fused signals.

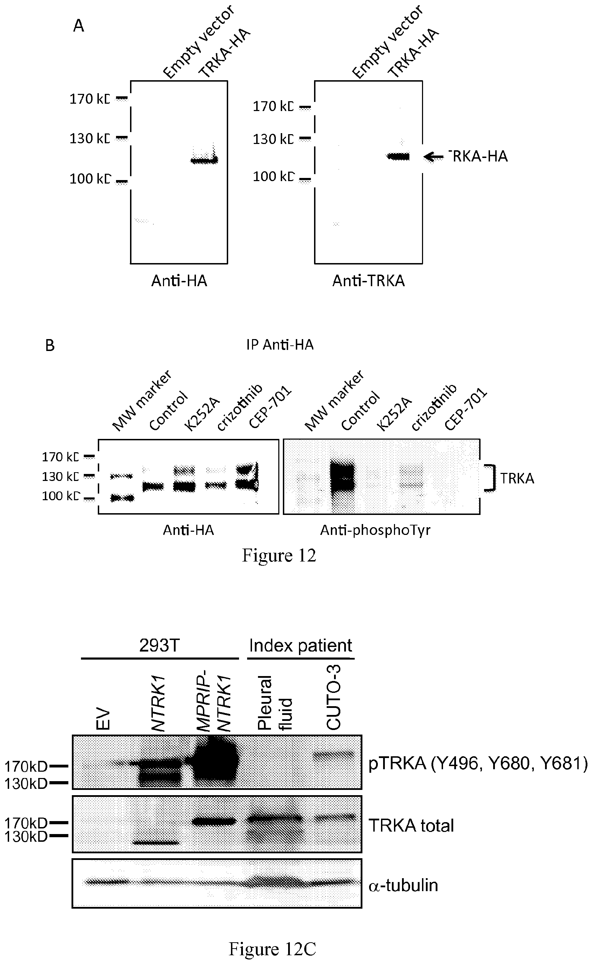

Figure 12A and B show immunoblot analyses of cell lysates from 293T cells expressing TRKA. Figure 12A, Expression of TRKA (with HA tag) or empty vector demonstrates expression of a ∼115-120kD protein detected by an HA-specific antibody (left, Cell Signaling) and a TRKA-specific antibody (right, Santa Cruz, SC-118). Figure 12B, Immunoprecipation using an HA-specific antibody (Cell Signaling) followed by immunoblot using the same antibody (left) or a phosphotyrosine specific antibody (right, Miilipore, 4G10) following treatment with 1 µM of the indicated inhibitors or DMSO (control) for 5 hours. Figure 12C shows the expression of NTRK1-MPRIP yields a chimeric protein that is autophosphorylated. Immunoblot analysis of 293T cells transiently transfected with empty vector (EV), full length NTRK1 cDNA, NTRK1-MPRIP cDNA compared to tumor cells from a frozen pleural fluid sample or early passage cells in culture (CUTO-3) from the index patient with the NTRK1-MPRIP fusion gene. Figure 13D is a schematic demonstrating fusion break-point and critical domains of predicted fusion protein products.

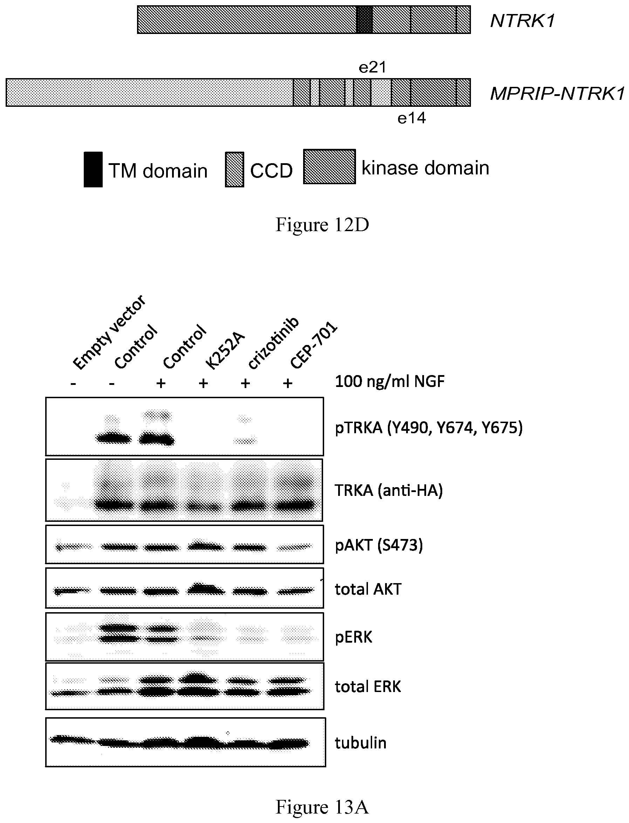

Figure 13A shows immunoblot analyses of downstream signalling of TRKA following treatment with tyrosine kinase inhibitors. SDS-PAGE of 293T cell lysates with expression of TRKA-HA or empty vector in the presence or absence of NGF (10 minutes) and the presence or absence of the indicated tyrosine kinase inhibitors at 1 µM for 5 hours. Membranes were probed with antibodies to TRKA phosphotyrosine 490, 674, and 675 (Cell Signaling), total TRKA (anti-HA, Cell Signaling), AKT phosphoserine 473 (Cell Signaling), total AKT (Cell Signaling), phosphorylated ERK p42/44 (Cell Signaling), total ERK p42/44 (Cell Signaling), and gamma-tubulin (Santa Cruz, SC-8035).

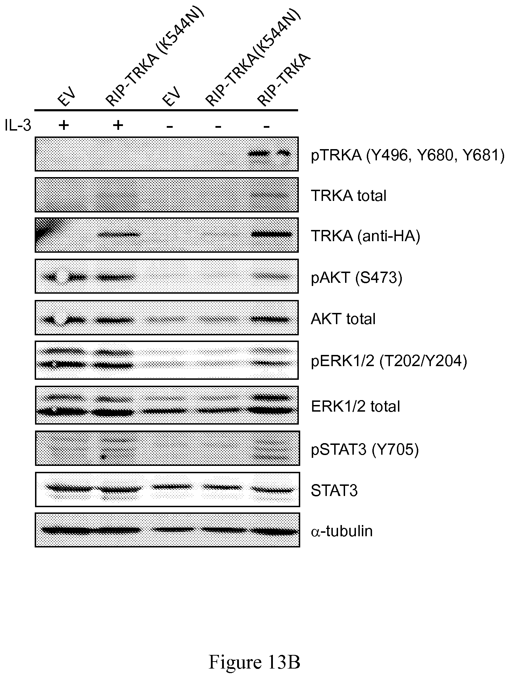

Figure 13B shows the expression of NTRK1-MPRIP induces activation of downstream MAPK, AKT, and STAT3 pathways. TRKA (NTRK1) fusions are autophosphorylated and activate key downstream signaling pathways. Representative immunoblot analyses (n = 3) of cell lysates from Ba/F3 cells expressing RIP-TRKA, the protein product of NTRK1-MPRIP but not its kinase dead (KD) variant display phosphorylation of critical tyrosine residues and activation of pAKT, pERK and pSTAT3 in the absence of IL-3.

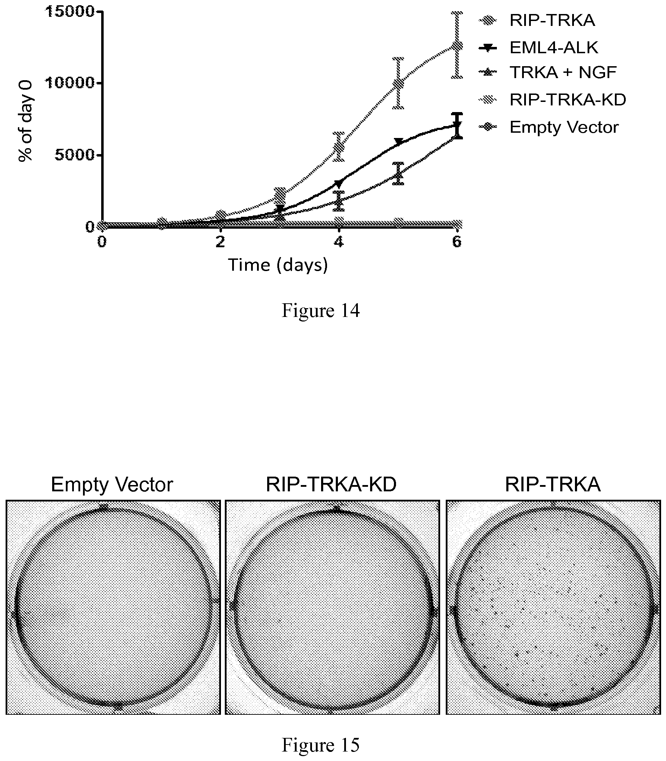

Figure 14 demonstrates that *NTRK1* gene fusions support cellular proliferation of Ba/F3 cells in the absence of IL-3. MTS assay of Ba/F3 demonstrates that cells expressing RIP-TRKA, CD74-TRKA, EML4-ALK, or full length TRKA supplemented with NGF proliferate in the absence of IL-3, whereas Ba/F3 cells expressing EV or the kinase dead variant of RIP-TRKA do not proliferate (*n* = 3). Values are mean ± SEM.
Figure 15 demonstrates that *NTRK1* fusions support anchorage independent growth. Representative images (*n* = 4) from anchorage independent growth assays of NIH3T3 cells expressing EV, RIP-TRKA-kinase dead (KD), or RIP-TRKA in soft agar.

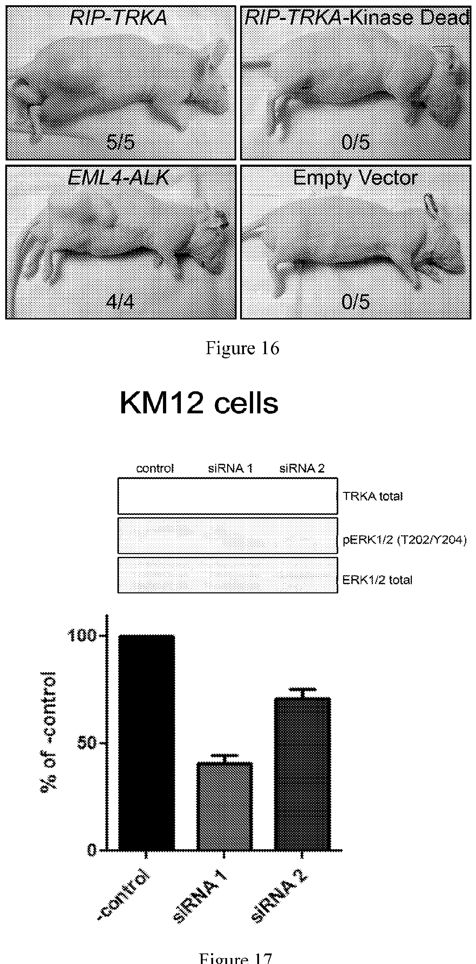

Figure 16 shows *NTRK1-MPRIP* fusion proteins induce tumorigenesis. NIH3T3 cells expressing *NTRK1-MPRIP* ("RIP-TRKA"), *NTRK1-MPRIP* kinase dead ("RIP-TRKA-Kinase Dead"), *EML4-ALK* or Empty Vector were injected into the flanks of nude mice and observed for tumor growth. The number of mice with tumors compared to the total number mice injected are indicated.
Figure 17 shows RNAi knockdown of *NTRK1* inhibits cell proliferation in a cell line harboring *TPM3-NTRK1.* KM12 cells were analyzed by MTS proliferation assay 96hr after siRNA transfection (*n* = 3). ANOVA analysis followed by Bonferroni's multiple comparison test indicated a significant inhibition of proliferation induced by siRNA 1 (p<0.05). Values represent the mean ± SEM. KM12 cells were transfected with siRNAs targeting *NTRK1* and then harvested 48hr later. Cell lysates were analyzed by immunoblot to detect TRKA, pERK1/2 and ERK1/2.

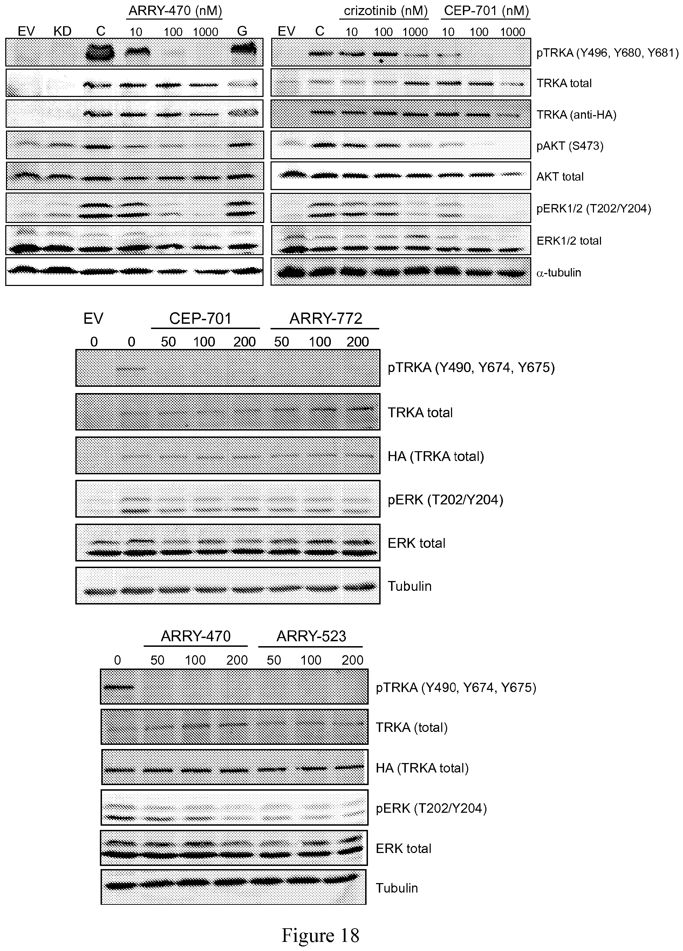

Figure 18 shows drug inhibition of activation of TRKA and downstream signaling. Ba/F3 cells expressing *NTRK1-MPRIP* (RIP-TRKA) or empty vector (EV) were lysed after 5h of treatment with the indicated doses of drugs (ARRY-470, crizotinib, CEP-701, ARRY-772, or ARRY-523) or DMSO control (C).

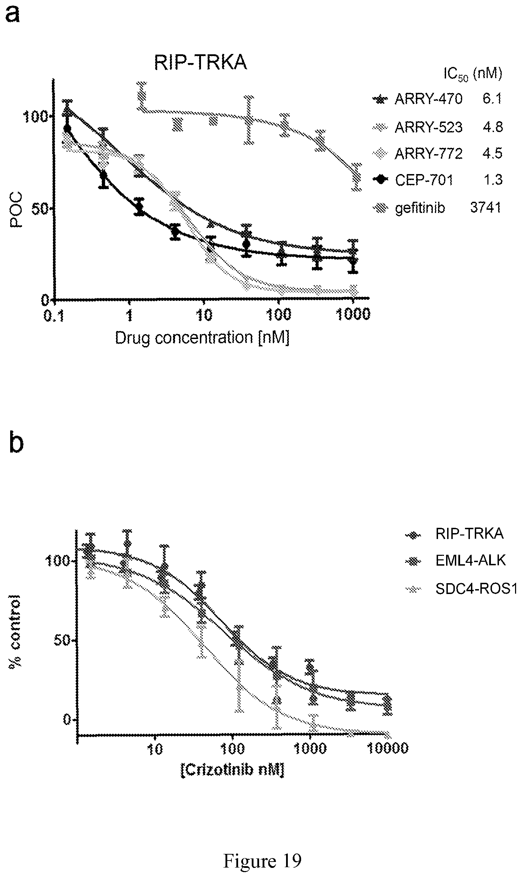

Figure 19 shows that drug treatment inhibits *NTRK1* fusion-mediated Ba/F3 cell proliferation and shows the treatment of index patient with crizotinib. Treatment of Ba/F3 cells expressing *NTRK1* fusions with TRKA inhibitors inhibits cell proliferation as measured by MTS assay (*n* = 5). Figure 19(a) Values represent the mean ± SEM. Ba/F3 cells expressing *NTRK1-MPRIP* demonstrate inhibition of proliferation by the pan-TRK inhibitors, ARRY-470, -523, and - 772 and the multi-kinase inhibitor, CEP-701, but not the EGFR inhibitor, gefitinib. Figure 19(b) Crizotinib leads to inhibition of Ba/F3 expressing *NTRK1* fusions, similar to Ba/F3 cells expressing ALK or ROS1 fusion constructs. The half maximal inhibitory concentration (IC₅₀) values are listed (nM).

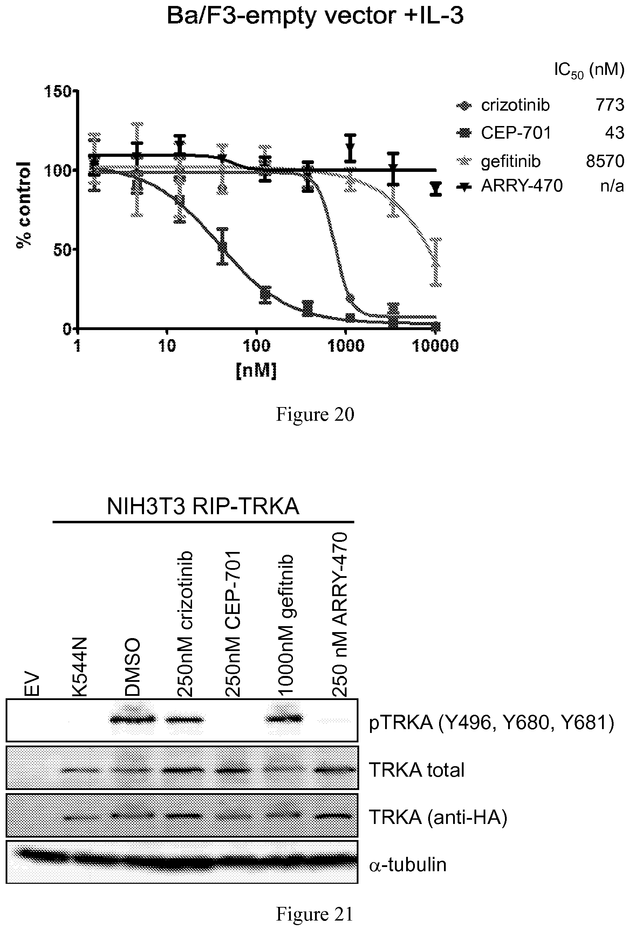

Figure 20 shows the drug treatment of Ba/F3 cells in the presence of IL-3. Ba/F3 cells expressing empty vector were grown in the presence of IL-3 and treated with a range of doses of ARRY-470, CEP-701, crizotinib, or gefitinib. IC₅₀ values are listed (*n* = 3). Values represent the mean ± SEM.
Figure 21 shows the expression and drug inhibition of *NTRK1* fusions in NIH3T3 cells. NIH3T3 cells expressing RIP-TRKA were treated with the indicated doses of drugs for 5h prior to cell lysis and immunoblot analysis of pTRKA, TRKA, pAKT, AKT, pERK1/2, ERK1/2, pSTAT3, and STAT3 as indicated.

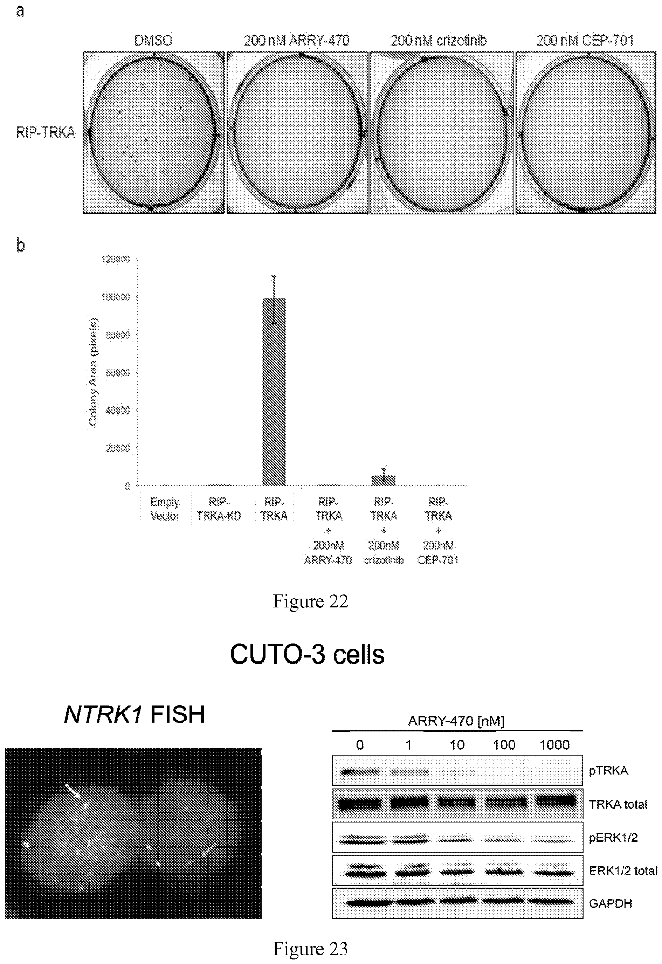

Figure 22 shows the inhibition of anchorage-independent growth by drugs with TRKA activity. Figure 22a, NIH3T3 cells expressing empty RIP-TRKA were seeded in triplicate in soft agar and treated with DMSO (control) or 200nM of ARRY-470, crizotinib, or CEP-701 for 2 weeks (*n* = 4). Representative images are shown. Figure 22b, The total colony area for each plate was quantified using MetaMorph software and plotted for each condition. Values represent the mean ± SEM.
Figure 23 shows the short term cell culture from index patient showing the NTRK1-MPRIP fusion. Colorado University Thoracic Oncology (CUTO) 3 cells were derived from a pleural effusion from the index patient harboring the NTRK1-MPRIP gene fusion. Left: *NTRK1* FISH analysis of CUTO-3 cells showing a positive signal (split green/red signals). Right: Immunoblot analysis of CUTO-3 cells demonstrating inhibition of pTRKA and pERK by the pan-TRK inhibitor, ARRY-470.

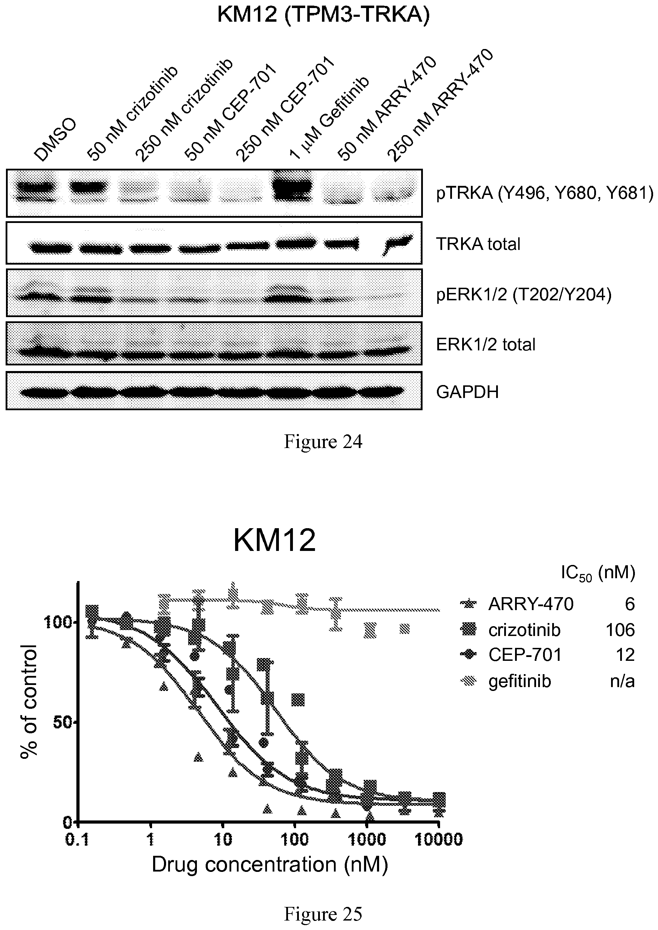

Figure 24 shows drug treatment of KM12 cells. KM12 cells harboring the *TPM3-NTRK1* fusion were lysed following 5h treatment with the indicated doses of inhibitors and subject to immunoblot analysis (*n* = 3).
Figure 25 shows that drug treatment of KM12 cells inhibits proliferation. Proliferation of KM12 cells treated with the indicated drugs and doses were assayed for cell proliferation by MTS assay. KM12 cells are inhibited by ARRY-470, CEP-701, and crizotinib, but not gefitinib.

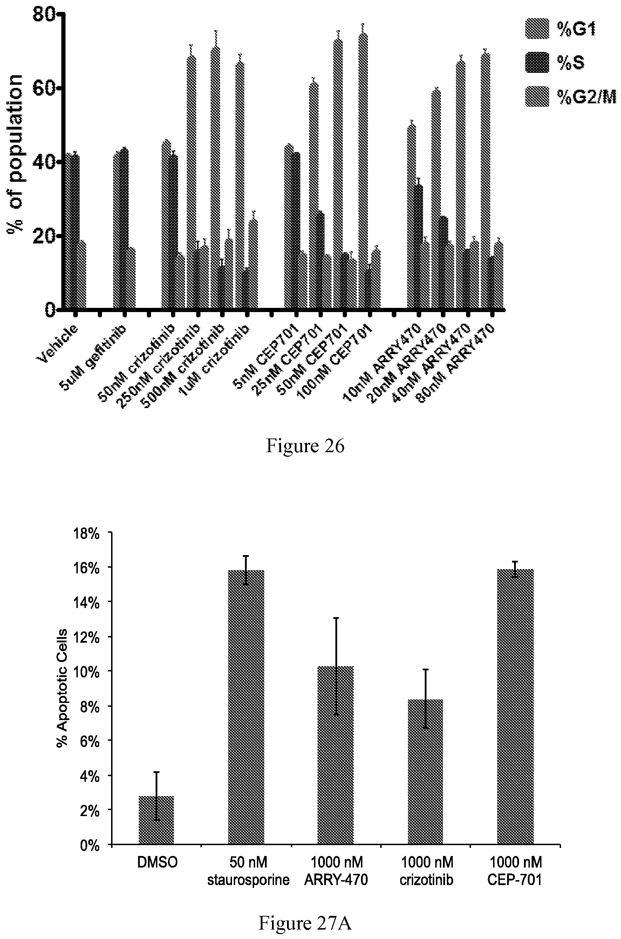

Figure 26 shows that TRKA inhibition results in the accumulation of KM12 cells in G1 phase. KM12 cells were treated with the indicated doses of drugs for 24hr. Cells were then stained with propidium iodide and analyzed by flow cytometry. ModFit analysis was used to quantify cell cycle profiles (*n* = 3). Values are the mean ± SEM.

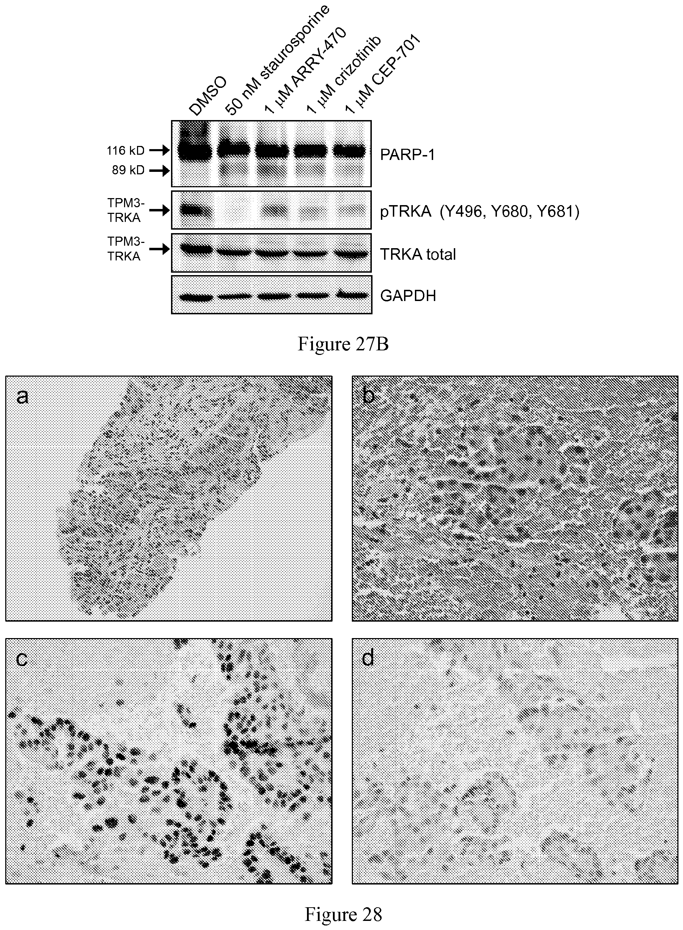

Figure 27 shows that drug treatment with TRKA inhibitors induces apoptosis in KM12 cells. Figure 27A, KM12 cells were treated for 24h with the indicated drugs and doses, trypsinized, stained with YO-PRO® and propidium iodide (PI), and analyzed by flow-cytometry. The percent of cells undergoing apoptosis (YO-PRO® positive and PI negative) are plotted (*n* = 4). Values represent the mean ± SEM. Figure 27B TRKA inhibitors induce cleavage of PARP-1. KM12 cells were treated for 24h with the indicated drugs and doses. Cells were lysed, separated by SDS-PAGE and subject to immunoblot analysis with the indicated antibodies.
Figure 28 shows histopathology from index patient harboring *NTRK1-MPRIP* demonstrating lung adenocarcinoma. Figure 28 (a): Needle core biopsy of primary lung left lower lung mass showing adenocarcinoma. Figure 28(b): Cell block of fine needle aspirate from the same procedure showing tumor cells. Figure 28(c): TTF-1 immunohistochemistry (IHC) demonstrating strong nuclear staining in tumor cells. Figure 28(d): Thyrogloblin IHC demonstrating negative staining in tumor cells. Representative images are shown.

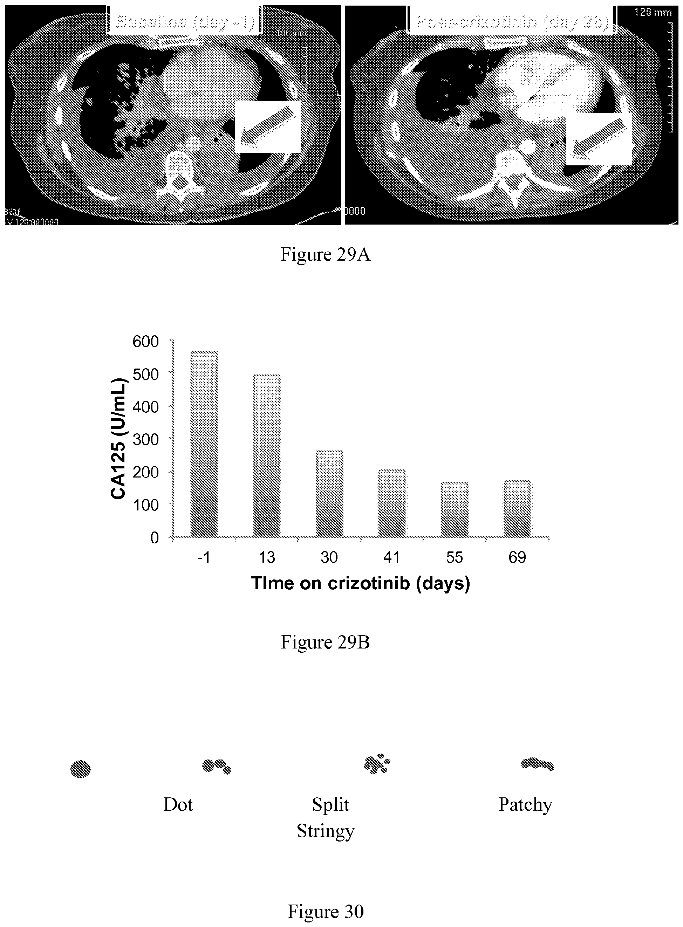

Figure 29 shows the results of treatment when the index patient (*NTRK1-MPRIP*) consented to treatment with crizotinib 250mg PO BID (off-protocol, off-label) given lack of other therapeutic options. Figure 29A: CT scan of the chest before and after 28d of crizotinib and Figure 29B: serial CA125 tumor marker levels during crizotinib treatment.
Figure 30 shows the signal configuration "Dot," which is the typical round and compact signal and comparison with other signals.

### DETAILED DESCRIPTION OF THE DISCLOSURE

The present inventors have discovered that fusion of the *MPRIP* and *NTRK1* genes is indicative of the presence of a specific type of lung cancer. This gene fusion is also indicative of patient clinical response to treatment with tyrosine kinase inhibitors. This gene fusion, and the levels of the protein encoded by this gene fusion, along with clinical parameters can be used as biological markers to diagnose a specific type of lung cancer and to assess cancer patient response to treatment with tyrosine kinase inhibitors.

According to one definition, a biological marker is "a characteristic that is objectively measured and evaluated as an indicator of normal biologic processes, pathogenic processes, or pharmacological responses to therapeutic interventions." NIH Marker Definitions Working Group (1998). Biological markers can also include patterns or ensembles of characteristics indicative of particular biological processes ("panel of markers"). The marker measurement can be increased or decreased to indicate a particular biological event or process. In addition, if a marker measurement typically changes in the absence of a particular biological process, a constant measurement can indicate occurrence of that process.

Marker measurements may be of the absolute values (e.g., the molar concentration of a molecule in a biological sample) or relative values (e.g., the relative concentration of two molecules in a biological sample). The quotient or product of two or more measurements also may be used as a marker. For example, some physicians use the total blood cholesterol as a marker of the risk of developing coronary artery disease, while others use the ratio of total cholesterol to HDL cholesterol.

In the present invention, the markers may be used for diagnostic, prognostic, therapeutic, drug screening and patient stratification purposes (e.g., to group patients into a number of "subsets" for evaluation), as well as other purposes described herein, including evaluation of the effectiveness of a potential cancer therapeutic.

The practice of the invention employs, unless otherwise indicated, conventional methods of analytical biochemistry, microbiology, molecular biology and recombinant DNA generally known techniques within the skill of the art. Such techniques are explained fully in the literature. (See, e.g., Sambrook et al. Molecular Cloning: A Laboratory Manual. 3rd, ed., Cold Spring Harbor Laboratory, Cold Spring Harbor Laboratory Press, Cold Spring Harbor, NY, 2000; DNA Cloning: A Practical Approach, Vol. I & II (Glover, ed.); Oligonucleotide Synthesis (Gait, ed., Current Edition); Nucleic Acid Hybridization (Hames & Higgins, eds., Current Edition); Transcription and Translation (Hames & Higgins, eds., Current Edition); CRC Handbook of Parvoviruses, Vol. I & II (Tijessen, ed.); Fundamental Virology, 2nd Edition, Vol. I & II (Fields and Knipe, eds.)).

The terminology used herein is for describing particular embodiments and is not intended to be limiting. As used herein, the singular forms "a," "and" and "the" include plural referents unless the content and context clearly dictate otherwise. Thus, for example, a reference to "a marker" includes a combination of two or more such markers. Unless defined otherwise, all scientific and technical terms are to be understood as having the same meaning as commonly used in the art to which they pertain. For the purposes of the present invention, the following terms are defined below.

As used herein, the term "marker" includes polypeptide markers and polynucleotide markers. For clarity of disclosure, aspects of the invention will be described with respect to "polypeptide markers" and "polynucleotide markers." However, statements made herein with respect to "polypeptide markers" are intended to apply to other polypeptides of the invention. Likewise, statements made herein with respect to "polynucleotide" markers are intended to apply to other polynucleotides of the invention, respectively. Thus, for example, a polynucleotide described as encoding a "polypeptide marker" is intended to include a polynucleotide that encodes: a polypeptide marker, a polypeptide that has substantial sequence identity to a polypeptide marker, modified polypeptide markers, fragments of a polypeptide marker, precursors of a polypeptide marker and successors of a polypeptide marker, and molecules that comprise a polypeptide marker, homologous polypeptide, a modified polypeptide marker or a fragment, precursor or successor of a polypeptide marker (e.g., a fusion protein).

As used herein, the term "polypeptide" refers to a polymer of amino acid residues that has at least 5 contiguous amino acid residues, e.g., 5, 6, 7, 8, 9, 10, 11 or 12 or more amino acids long, including each integer up to the full length of the polypeptide. A polypeptide may be composed of two or more polypeptide chains. A polypeptide includes a protein, a peptide, an oligopeptide, and an amino acid. A polypeptide can be linear or branched. A polypeptide can comprise modified amino acid residues, amino acid analogs or non-naturally occurring amino acid residues and can be interrupted by non-amino acid residues. Included within the definition are amino acid polymers that have been modified, whether naturally or by intervention, e.g., formation of a disulfide bond, glycosylation, lipidation, methylation, acetylation, phosphorylation, or by manipulation, such as conjugation with a labeling component. Also included are antibodies produced by a subject in response to overexpressed polypeptide markers.

As used herein, a "fragment" of a polypeptide refers to a single amino acid or a plurality of amino acid residues comprising an amino acid sequence that has at least 5 contiguous amino acid residues, at least 10 contiguous amino acid residues, at least 20 contiguous amino acid residues or at least 30 contiguous amino acid residues of a sequence of the polypeptide. As used herein, a "fragment" of polynucleotide refers to a single nucleic acid or to a polymer of nucleic acid residues comprising a nucleic acid sequence that has at least 15 contiguous nucleic acid residues, at least 30 contiguous nucleic acid residues, at least 60 contiguous nucleic acid residues, or at least 90% of a sequence of the polynucleotide. In some embodiment, the fragment is an antigenic fragment, and the size of the fragment will depend upon factors such as whether the epitope recognized by an antibody is a linear epitope or a conformational epitope. Thus, some antigenic fragments will consist of longer segments while others will consist of shorter segments, (e.g. 5, 6, 7, 8, 9, 10, 11 or 12 or more amino acids long, including each integer up to the full length of the polypeptide). Those skilled in the art are well versed in methods for selecting antigenic fragments of proteins.

In some embodiments, a polypeptide marker is a member of a biological pathway. As used herein, the term "precursor" or "successor" refers to molecules that precede or follow the polypeptide marker or polynucleotide marker in the biological pathway. Thus, once a polypeptide marker or polynucleotide marker is identified as a member of one or more biological pathways, the present invention can include additional precursor or successor members of the biological pathway. Such identification of biological pathways and their members is within the skill of one in the art.

As used herein, the term "polynucleotide" refers to a single nucleotide or a polymer of nucleic acid residues of any length. The polynucleotide may contain deoxyribonucleotides, ribonucleotides, and/or their analogs and may be double-stranded or single stranded. A polynucleotide can comprise modified nucleic acids (e.g., methylated), nucleic acid analogs or non-naturally occurring nucleic acids and can be interrupted by non-nucleic acid residues. For example a polynucleotide includes a gene, a gene fragment, cDNA, isolated DNA, mRNA, tRNA, rRNA, isolated RNA of any sequence, recombinant polynucleotides, primers, probes, plasmids, and vectors. Included within the definition are nucleic acid polymers modified either naturally, or by intervention.

As used herein, a component (e.g., a marker) is referred to as "differentially expressed" in one sample as compared to another sample when the method used for detecting the component provides a different level or activity when applied to the two samples. A component is referred to as "increased" in the first sample if the method for detecting the component indicates that the level or activity of the component is higher in the first sample than in the second sample (or if the component is detectable in the first sample but not in the second sample). Conversely, a component is referred to as "decreased" in the first sample if the method for detecting the component indicates that the level or activity of the component is lower in the first sample than in the second sample (or if the component is detectable in the second sample but not in the first sample). In particular, marker is referred to as "increased" or "decreased" in a sample (or set of samples) obtained from a lung cancer subject (or a subject who is suspected of having lung cancer, or is at risk of developing lung cancer) if the level or activity of the marker is higher or lower, respectively, compared to the level of the marker in a sample (or set of samples) obtained from a non-lung cancer subject, or a reference value or range.

The novel gene fusion marker of the present invention was identified as follows: the presence of an oncogene driver gene abnormality was investigated in a non-smoker patient with lung adenocarcinoma. The patient showed no evidence of known mutations, gene amplifications or gene fusions associated with lung cancer. In order to pursue other possible gene targets, genomic DNA from a tumor biopsy sample was analyzed by targeted next generation sequencing, which identified the presence of a novel *NTRK1* gene fusion. The gene fusion marker was determined to be a *NTRK1-MPRIP* gene fusion.

The *NTRK1* gene encodes the TRKA receptor tyrosine kinase. The *NTRK1* gene has been isolated from a number of species such as human, chimpanzee, dog, cow, mouse, rat, chicken and zebrafish and the sequence determined. All these gene sequences are known to one skilled in the art and are intended to be encompassed in the present invention. Gene fusions involving *NTRK1* have previously been reported in papillary thyroid cancer, but have not been reported in lung cancer or other malignancies.

The *MPRIP* gene encodes the Myosin phosphatase Rho-interacting Protein. The *MPRIP* gene has been isolated from a number of species such as human, chimpanzee, dog, cow, mouse, rat, chicken, zebrafish and *C*. *elegans* and the sequence determined. All these gene sequences are known to one skilled in the art and are intended to be encompassed in the present invention.

This is believed to be the first instance identifying the *NTRK1-MPRIP* gene fusion in any malignancy. Customized RT-PCR assays, including novel primers, were developed to detect the mRNA transcript of the *NTRK1-MPRIP* gene fusion. The RT-PCR successfully amplified a small product containing sequences from both *MPRIP* and *NTRK1*, confirming expression of a novel gene fusion that included exon 1-21 of *MPRIP* and exons 14-20 of *NTRK1.* (See Example 1.) Novel FISH assays were also developed to detect the presence of the *NTRK1-MPRIP* gene fusion in clinical specimens. (See Examples 2 and 3.)

Additionally, FISH probes that would detect other *NTRK1* gene fusions, regardless of the specific 5' gene fusion partner, were also developed. (See Examples 2 and 4.)

The markers identified herein are of significant biologic interest. Gene fusions involving *NTRK1* have previously been reported in papillary thyroid cancer, but have not been reported in lung cancer or other malignancies. Thus, the *NTRK1* fusion gene serves as a novel diagnostic marker of cancer. *NTRK1* gene encodes the TRKA receptor tyrosine kinase. The presence of the gene fusion was examined in tumor samples obtained from various cancer models, including lung and colorectal cancers, and the sensitivity of the tumor to tyrosine kinase inhibitors was investigated. The objective was to use this gene fusion marker to identify a clinically relevant marker of cancer patient response to tyrosine kinase inhibitor treatment. The methods used are detailed in the Examples section of this disclosure. Several tyrosine kinase inhibitors that are currently in various stages of clinical development including, without limitation, crizotinib, ponatinib, dovitinib, rebastinib, CEP-701, AZD-7451, ARRY-470, ARRY-523, and ARRY-772 as well as other tyrosine kinase inhibitor compounds known in the art that are predicted to inhibit TRKA or oncogenic fusion proteins that contain the TRKA kinase domain, such as *NTRK1* fusion proteins. Data presented in Example 5 demonstrates that small molecule tyrosine kinase inhibitors inhibit activated TRKA.

In addition to the discovery of the *NTRK1* gene fusion marker that can be used for the diagnosis of, prognosis of, or other evaluation or study of cancer, the marker may also be studied in more detail and/or be used as target for the discovery of other modulators of disease or therapeutic agents.

It is believed that the *NTRK1* gene fusion markers, including the *NTRK1-MPRIP* gene fusion marker, are indicators of cancer patient response to tyrosine kinase inhibitors. Accordingly, in one aspect, the invention provides a marker, the presence or expression level of which is indicative of cancer patient response to tyrosine kinase inhibitors.

In another aspect, the gene fusion markers of the present invention can serve as indicators of cancer patient response to other targeted cancer therapies such as administration of HSP90 inhibitors (or other chaperone inhibitors) or agents that target downstream signalling cascades. Such inhibitors are well known in the art and are commercially available. All such inhibitors are encompassed in the present invention. Examples of HSP90 inhibitors include without limitation geldanamycin, herbimycin, 17-AAG, PU24FCl, STA-9090, IPI-504, and AUY-922. Examples of agents that target downstream signalling cascades include selumetinib (AZD-6244) and MK2206.

The presence of the marker may be detected by detecting a polynucleotide. In one embodiment, the polynucleotide may be a probe that specifically hybridizes with the *NTRK1* gene sequences and identifies a chromosomal rearrangement involving the *NTRK1* gene. In another embodiment, the polynucleotide may be a primer that specifically binds and amplifies a polynucleotide sequence that is indicative of the presence of the gene fusion involving a *NTRK1* gene, including the *NTRK1-MPRIP* gene fusion marker.

Some variation is inherent in the measurements of the physical and chemical characteristics of the markers of the invention. The magnitude of the variation depends to some extent on the reproducibility of the separation means and the specificity and sensitivity of the detection means used to make the measurement. Preferably, the method and technique used to measure the markers is sensitive and reproducible.

The presence of the gene fusion marker may also be detected by detecting a polynucleotide. Polypeptides corresponding to the *NTRK1* gene fusion markers may include a fragment, precursor, successor or modified version of the protein encoded by the *NTRK1*- gene fusion markers. In another embodiment, the invention includes a molecule that comprises a fragment, precursor, successor or modified polypeptide encoded by the *NTRK1*- gene fusion markers.

Another embodiment of the present invention relates to an assay system including a plurality of antibodies, or antigen binding fragments thereof, or aptamers for the detection of the expression of the *NTRK1* gene fusion markers of the invention. The plurality of antibodies, or antigen binding fragments thereof, or aptamers selectively bind to proteins encoded by the *NTRK1* gene fusion markers.

As used herein, the terms "patient," "subject," "a subject who has cancer" and "cancer patient" are intended to refer to subjects who have been diagnosed with a cancer or are suspected of having cancer. The terms "non-subject" and "a subject who does not have cancer" are intended to refer to a subject who has not been diagnosed with cancer, or who is cancer-free as a result of surgery to remove one or more tumors. A non-cancer subject may be healthy and have no other disease, or they may have a disease other than cancer. The *NTRK1* gene has been found to be conserved in a number of species such as chimpanzee, dog, cow, mouse, rat, chicken, and zebrafish and their sequences are known. In some embodiments, the patient or subject may be a mammal. In a preferred embodiment, the patient or subject is human.

Polypeptides encoded by the *NTRK1* gene fusion may be isolated by any suitable method known in the art. Native polypeptides encoded by the *NTRK1* gene fusion can be purified from natural sources by standard methods known in the art (e.g., chromatography, centrifugation, differential solubility, immunoassay). In one embodiment, the polypeptides may be isolated from a tumor sample. In another embodiment, the polypeptides may be isolated from a sample by contacting the sample with substrate-bound antibodies or aptamers that specifically bind to the marker.

The present invention also includes polynucleotides related to the gene fusion markers of the present invention. In one aspect, the invention provides polynucleotides that comprise the *NTRK1* gene fusion markers of the invention. These may be referred to as polynucleotide markers. The polynucleotide markers may be genomic DNA, cDNA, or mRNA transcripts. In another embodiment, the invention provides polynucleotides that have substantial sequence similarity to a polynucleotide that comprises the *NTRK1* gene fusion markers or variants thereof, including the *NTRK1*- gene fusion markers.

In some embodiments, the polypeptides encoded by the *NTRK1* gene fusion markers i.e. polypeptide markers may be used as surrogate markers of the *NTRK1-MPRIP* gene fusion. Thus, for example, if a polypeptide encoded by the *NTRK1-MPRIP* gene fusion markers is present in cancer patients, the presence or level or activity of the polypeptides may be interrogated (e.g., to identify cancer patients expected to respond to tyrosine kinase inhibitors).

Polynucleotide markers comprising the gene fusion markers may be isolated by any suitable method known in the art. Native polynucleotide markers may be purified from natural sources by standard methods known in the art (e.g., chromatography, centrifugation, differential solubility, immunoassay). In one embodiment, a polynucleotide marker may be isolated from a mixture by contacting the mixture with substrate bound probes that are complementary to the polynucleotide marker under hybridization conditions.

Alternatively, polynucleotide markers comprising the *NTRK1* gene fusion may be synthesized by any suitable chemical or recombinant method known in the art. In one embodiment, for example, the makers can be synthesized using the methods and techniques of organic chemistry. In another embodiment, a polynucleotide marker can be produced by polymerase chain reaction (PCR).

The present invention also encompasses molecules which specifically bind the polypeptide or polynucleotide markers of the present invention. In one aspect, the invention provides molecules that specifically bind to a polypeptide marker or a polynucleotide marker. As used herein, the term "specifically binding," refers to the interaction between binding pairs (e.g., an antibody and an antigen or aptamer and its target). In some embodiments, the interaction has an affinity constant of at most 10⁻⁶ moles/liter, at most 10⁻⁷ moles/liter, or at most 10⁻⁸ moles/liter. In other embodiments, the phrase "specifically binds" refers to the specific binding of one protein to another (*e*.*g*., an antibody, fragment thereof, or binding partner to an antigen), wherein the level of binding, as measured by any standard assay (*e*.*g*., an immunoassay), is statistically significantly higher than the background control for the assay. For example, when performing an immunoassay, controls typically include a reaction well/tube that contain antibody or antigen binding fragment alone (*i*.*e*., in the absence of antigen), wherein an amount of reactivity (*e*.*g*., nonspecific binding to the well) by the antibody or antigen binding fragment thereof in the absence of the antigen is considered to be background. Binding can be measured using a variety of methods standard in the art including enzyme immunoassays (*e*.*g*., ELISA), immunoblot assays, etc.).

The binding molecules include antibodies, aptamers and antibody fragments. As used herein, the term "antibody" refers to an immunoglobulin molecule capable of binding an epitope present on an antigen. The term is intended to encompasses not only intact immunoglobulin molecules such as monoclonal and polyclonal antibodies, but also bi-specific antibodies, humanized antibodies, chimeric antibodies, anti-idiopathic (anti-ID) antibodies, single-chain antibodies, Fab fragments, F(ab') fragments, fusion proteins and any modifications of the foregoing that comprise an antigen recognition site of the required specificity. As used herein, an aptamer is a non-naturally occurring nucleic acid having a desirable action on a target. A desirable action includes, but is not limited to, binding of the target, catalytically changing the target, reacting with the target in a way which modifies/alters the target or the functional activity of the target, covalently attaching to the target as in a suicide inhibitor, facilitating the reaction between the target and another molecule. In a preferred embodiment, the action is specific binding affinity for a target molecule, such target molecule being a three dimensional chemical structure other than a polynucleotide that binds to the nucleic acid ligand through a mechanism which predominantly depends on Watson/Crick base pairing or triple helix binding, wherein the nucleic acid ligand is not a nucleic acid having the known physiological function of being bound by the target molecule.

Certain antibodies that specifically bind polypeptide markers polynucleotide markers of the invention already may be known and/or available for purchase from commercial sources. In any event, the antibodies of the invention may be prepared by any suitable means known in the art. For example, antibodies may be prepared by immunizing an animal host with a marker or an immunogenic fragment thereof (conjugated to a carrier, if necessary). Adjuvants (e.g., Freund's adjuvant) optionally may be used to increase the immunological response. Sera containing polyclonal antibodies with high affinity for the antigenic determinant can then be isolated from the immunized animal and purified.

Alternatively, antibody-producing tissue from the immunized host can be harvested and a cellular homogenate prepared from the organ can be fused to cultured cancer cells. Hybrid cells which produce monoclonal antibodies specific for a marker can be selected. Alternatively, the antibodies of the invention can be produced by chemical synthesis or by recombinant expression. For example, a polynucleotide that encodes the antibody can be used to construct an expression vector for the production of the antibody. The antibodies of the present invention can also be generated using various phage display methods known in the art.

Antibodies or aptamers that specifically bind markers of the invention can be used, for example, in methods for detecting protein products encoded by the *NTRK1* gene fusion markers of the invention. In one embodiment, antibodies or aptamers against a polypeptide marker or polynucleotide marker of the invention can be used to assay a tissue sample (e.g., a thin cortical slice) for the markers. The antibodies or aptamers can specifically bind to the marker, if any, present in the tissue sections and allow the localization of the marker in the tissue. Similarly, antibodies or aptamers labelled with a radioisotope may be used for *in vivo* imaging or treatment applications.

The present invention also provides methods of detecting the *NTRK1* gene fusion markers of the present invention. The practice of the present invention employs, unless otherwise indicated, conventional methods of analytical biochemistry, microbiology, molecular biology and recombinant DNA techniques within the skill of the art. Such techniques are explained fully in the literature. (See, e.g., Sambrook, J. et al. Molecular Cloning: A Laboratory Manual. 3rd, ed., Cold Spring Harbor Laboratory, Cold Spring Harbor Laboratory Press, Cold Spring Harbor, NY, 2000; DNA Cloning: A Practical Approach, Vol. I & II (D. Glover, ed.); Oligonucleotide Synthesis (N. Gait, ed., Current Edition); Nucleic Acid Hybridization (B. Hames & S. Higgins, eds., Current Edition); Transcription and Translation (B. Hames & S. Higgins, eds., Current Edition); CRC Handbook of Parvoviruses, Vol. I & II (P. Tijessen, ed.); Fundamental Virology, 2nd Edition, Vol. I & II (B. N. Fields and D. M. Knipe, eds.)).

The markers of the invention may be detected by any method known to those of skill in the art, including without limitation LC-MS, GC-MS, immunoassays, hybridization and enzyme assays. The detection may be quantitative or qualitative. A wide variety of conventional techniques are available, including mass spectrometry, chromatographic separations, 2-D gel separations, binding assays (e.g., immunoassays), competitive inhibition assays, and so on. Any effective method in the art for measuring the presence/absence, level or activity of a polypeptide or polynucleotide is included in the invention. It is within the ability of one of ordinary skill in the art to determine which method would be most appropriate for measuring a specific marker. Thus, for example, an ELISA assay may be best suited for use in a physician's office while a measurement requiring more sophisticated instrumentation may be best suited for use in a clinical laboratory. Regardless of the method selected, it is important that the measurements be reproducible.

For protein markers, quantification can be based on derivatization in combination with isotopic labelling, referred to as isotope coded affinity tags ("ICAT"). In this and other related methods, a specific amino acid in two samples is differentially and isotopically labelled and subsequently separated from peptide background by solid phase capture, wash and release. The intensities of the molecules from the two sources with different isotopic labels can then be accurately quantified with respect to one another. Quantification can also be based on the isotope dilution method by spiking in an isotopically labelled peptide or protein analogous to those being measured. Furthermore, quantification can also be determined without isotopic standards using the direct intensity of the analyte comparing with another measurement of a standard in a similar matrix.

In addition, one- and two-dimensional gels have been used to separate proteins and quantify gels spots by silver staining, fluorescence or radioactive labelling. These differently stained spots have been detected using mass spectrometry, and identified by tandem mass spectrometry techniques.

A number of the assays discussed above employ a reagent that specifically binds to a *NTRK1* gene fusion marker of the invention. Any molecule that is capable of specifically binding to the *NTRK1* gene fusion markers of the invention is included within the invention. In some embodiments, the binding molecules are antibodies or antibody fragments. In other embodiments, the binding molecules are non-antibody species, such as aptamers or nucleotide probes.

As described above, the binding molecules may be identified and produced by any method accepted in the art. Methods for identifying and producing antibodies and antibody fragments specific for an analyte are well known.

The markers of the invention also may be detected or measured using a number of chemical derivatization or reaction techniques known in the art. Reagents for use in such techniques are known in the art, and are commercially available for certain classes of target molecules.

Measurement of the relative amount of an RNA or protein marker of the invention may be by any method known in the art (see, e.g., Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989; and Current Protocols in Molecular Biology, eds. Ausubel et al. John Wiley & Sons: 1992). Typical methodologies for RNA detection include RNA extraction from a cell or tissue sample, followed by hybridization of a labelled probe (e.g., a complementary polynucleotide) specific for the target RNA to the extracted RNA, and detection of the probe (e.g., Northern blotting). Typical methodologies for protein detection include protein extraction from a cell or tissue sample, followed by hybridization of a labelled probe (e.g., an antibody) specific for the target protein to the protein sample, and detection of the probe. The label group can be a radioisotope, a fluorescent compound, an enzyme, or an enzyme co-factor. Detection of specific protein and polynucleotides may also be assessed by gel electrophoresis, column chromatography, direct sequencing, or quantitative PCR (in the case of polynucleotides) among many other techniques well known to those skilled in the art.

Detection of the presence or number of copies of all or a part of a marker gene of the invention may be performed using any method known in the art. Typically, it is convenient to assess the presence and/or quantity of a DNA or cDNA by Southern analysis, in which total DNA from a cell or tissue sample is extracted, is hybridized with a labelled probe (e.g., a complementary DNA molecule), and the probe is detected. The label group can be a radioisotope, a fluorescent compound, an enzyme, or an enzyme co-factor. Other useful methods of DNA detection and/or quantification include direct sequencing, gel electrophoresis, column chromatography, and quantitative PCR, as is known by one skilled in the art.

Polynucleotide similarity can be evaluated by hybridization between single stranded nucleic acids with complementary or partially complementary sequences. Such experiments are well known in the art. High stringency hybridization and washing conditions, as referred to herein, refer to conditions which permit isolation of nucleic acid molecules having at least about 80% nucleic acid sequence identity with the nucleic acid molecule being used to probe in the hybridization reaction (i.e., conditions permitting about 20% or less mismatch of nucleotides). Very high stringency hybridization and washing conditions, as referred to herein, refer to conditions which permit isolation of nucleic acid molecules having at least about 90% nucleic acid sequence identity with the nucleic acid molecule being used to probe in the hybridization reaction (i.e., conditions permitting about 10% or less mismatch of nucleotides). One of skill in the art can calculate the appropriate hybridization and wash conditions to achieve these particular levels of nucleotide mismatch. Such conditions will vary, depending on whether DNA:RNA or DNA:DNA hybrids are being formed. Calculated melting temperatures for DNA:DNA hybrids are 10°C less than for DNA:RNA hybrids. In particular embodiments, stringent hybridization conditions for DNA:DNA hybrids include hybridization at an ionic strength of 6X SSC (0.9 M Na⁺) at a temperature of between about 20°C and about 35°C (lower stringency), more preferably, between about 28°C and about 40°C (more stringent), and even more preferably, between about 35°C and about 45°C (even more stringent), with appropriate wash conditions. In particular embodiments, stringent hybridization conditions for DNA:RNA hybrids include hybridization at an ionic strength of 6X SSC (0.9 M Na⁺) at a temperature of between about 30°C and about 45°C, more preferably, between about 38°C and about 50°C, and even more preferably, between about 45°C and about 55°C, with similarly stringent wash conditions. These values are based on calculations of a melting temperature for molecules larger than about 100 nucleotides, 0% formamide and a G + C content of about 40%. Alternatively, Tₘ can be calculated empirically as set forth in Sambrook et al., *supra*, pages 9.31 to 9.62. In general, the wash conditions should be as stringent as possible, and should be appropriate for the chosen hybridization conditions. For example, hybridization conditions can include a combination of salt and temperature conditions that are approximately 20-25°C below the calculated Tₘ of a particular hybrid, and wash conditions typically include a combination of salt and temperature conditions that are approximately 12-20°C below the calculated Tₘ of the particular hybrid. One example of hybridization conditions suitable for use with DNA:DNA hybrids includes a 2-24 hour hybridization in 6X SSC (50% formamide) at about 42°C, followed by washing steps that include one or more washes at room temperature in about 2X SSC, followed by additional washes at higher temperatures and lower ionic strength (e.g., at least one wash as about 37°C in about 0.1X-0.5X SSC, followed by at least one wash at about 68°C in about 0.1X-0.5X SSC). Other hybridization conditions, and for example, those most useful with nucleic acid arrays, will be known to those of skill in the art.

Using the methods of the present invention, administration of a chemotherapeutic drug or drug combination can be evaluated or re-evaluated in light of the assay results of the present invention. For example, the tyrosine kinase inhibitor drug(s) can be administered differently to different subject populations, depending on the presence of the *NTRK1-MPRIP* gene fusion markers of the invention in tumor samples from the subjects tested. Results from the different drug regimens can also be compared with each other directly. Alternatively, the assay results may indicate the desirability of one drug regimen over another, or indicate that a specific drug regimen should or should not be administered to a cancer patient. In one preferred embodiment, the finding of the presence of the *NTRK1-MPRIP* gene fusion markers of the invention is indicative of a good prognosis for response to treatment with chemotherapeutic agents comprising tyrosine kinase inhibitors ("tyrosine kinase inhibitor chemotherapeutic agents"). In another preferred embodiment, the absence of the *NTRK1-MPRIP* gene fusion markers of the invention in a cancer patient is indicative of a poor prognosis for response to treatment with tyrosine kinase inhibitor chemotherapeutic agents, and may further recommend not administering tyrosine kinase inhibitor chemotherapeutic agent drug regimens.

In another aspect, the invention relates to the use of a kit for identifying cancer patients predicted to respond or not respond to tyrosine kinase inhibitor drugs, based on the presence or absence of *NTRK1-MPRIP* gene fusion markers of the disclosure.

The kits for use according to the invention may comprise one or more of the following: an antibody, wherein the antibody specifically binds with a polypeptide marker, a labelled binding partner to the antibody, a solid phase upon which is immobilized the antibody or its binding partner, a polynucleotide probe that can hybridize to a polynucleotide marker, pairs of primers that under appropriate reaction conditions can prime amplification of at least a portion of a gene fusion polynucleotide marker (e.g., by PCR), instructions on how to use the kit, and a label or insert indicating regulatory approval for diagnostic or therapeutic use.

The disclosure further includes polynucleotide or polypeptide microarrays comprising polypeptides of the invention, polynucleotides of the invention, or molecules, such as antibodies, which specifically bind to the polypeptides or polynucleotides of the present invention. In this aspect of the invention, standard techniques of microarray technology are utilized to assess expression of the polypeptides markers and/or identify biological constituents that bind such polypeptides. Protein microarray technology is well known to those of ordinary skill in the art and is based on, but not limited to, obtaining an array of identified peptides or proteins on a fixed substrate, binding target molecules or biological constituents to the peptides, and evaluating such binding. Polynucleotide arrays, particularly arrays that bind polypeptides of the invention, also can be used for diagnostic applications, such as for identifying subjects that have a condition characterized by expression of polypeptide markers, e.g., cancer.

The assay systems of the present disclosure can include a means for detecting in a sample of tumor cells the presence of the *NTRK1* gene fusion markers of the invention, and/or a level of expression of the *NTRK1-MPRIP* gene fusion markers of the invention, and/or a level of protein product of the *NTRK1-MPRIP* gene fusion markers of the invention.

The assay system preferably also includes one or more controls. The controls may include: (i) a control sample for detecting sensitivity to tyrosine kinase inhibitor chemotherapeutics; (ii) a control sample for detecting resistance to tyrosine kinase inhibitor chemotherapeutics; (iii) information containing a predetermined control level of markers to be measured with regard to tyrosine kinase inhibitor sensitivity or resistance (e.g., a predetermined control level of a marker of the *NTRK1* gene fusion of the present invention that has been correlated with sensitivity to tyrosine kinase inhibitor chemotherapeutics or resistance to tyrosine kinase inhibitor chemotherapeutics).

In another aspect, a means for detecting the *NTRK1* gene fusion markers of the disclosure can generally be any type of reagent that can include, but are not limited to, polynucleotides, hybridization probes, PCR primers, antibodies and antigen binding fragments thereof, peptides, binding partners, aptamers, enzymes, and small molecules. Additional reagents useful for performing an assay using such means for detection can also be included, such as reagents for performing immunohistochemistry, Fluorescent *in situ* Hybridization (FISH) or a preferred binding assay.

The means for detecting of the assay system of the present disclosure can be conjugated to a detectable tag or detectable label. Such a tag can be any suitable tag which allows for detection of the reagents used to detect the gene or protein of interest and includes, but is not limited to, any composition or label detectable by spectroscopic, photochemical, electrical, optical or chemical means. Useful labels in the present invention include: biotin for staining with labeled streptavidin conjugate, magnetic beads (*e*.*g*., DYNABEADS™), fluorescent dyes (*e*.*g*., fluorescein, texas red, rhodamine, green fluorescent protein, and the like), radiolabels (*e*.*g*., ³H, ¹²⁵I, ³⁵S, ¹⁴C, or ³²P), enzymes (*e*.*g*., horse radish peroxidase, alkaline phosphatase and others commonly used in an ELISA), and colorimetric labels such as colloidal gold or colored glass or plastic (*e*.*g*., polystyrene, polypropylene, latex, etc.) beads.

In addition, the means for detecting of the assay system of the present disclosure can be immobilized on a substrate. Such a substrate can include any suitable substrate for immobilization of a detection reagent such as would be used in any of the previously described methods of detection. Briefly, a substrate suitable for immobilization of a means for detecting includes any solid support, such as any solid organic, biopolymer or inorganic support that can form a bond with the means for detecting without significantly affecting the activity and/or ability of the detection means to detect the desired target molecule. Exemplary organic solid supports include polymers such as polystyrene, nylon, phenol-formaldehyde resins, and acrylic copolymers (*e*.*g*., polyacrylamide). The kit can also include suitable reagents for the detection of the reagent and/or for the labeling of positive or negative controls, wash solutions, dilution buffers and the like. The assay system can also include a set of written instructions for using the system and interpreting the results.

The assay system can also include a means for detecting a control marker that is characteristic of the cell type being sampled can generally be any type of reagent that can be used in a method of detecting the presence of a known marker (at the nucleic acid or protein level) in a sample, such as by a method for detecting the presence of a marker described previously herein. Specifically, the means is characterized in that it identifies a specific marker of the cell type being analyzed that positively identifies the cell type. For example, in a lung tumor assay, it is desirable to screen lung cancer cells for the level of the marker expression and/or biological activity. Therefore, the means for detecting a control marker identifies a marker that is characteristic of, for example, a lung cell, so that the cell is distinguished from other cell types, such as a connective tissue or inflammatory cell. Such a means increases the accuracy and specificity of the assay of the present invention. Such a means for detecting a control marker include, but are not limited to: a probe that hybridizes under stringent hybridization conditions to a nucleic acid molecule encoding a protein marker; PCR primers which amplify such a nucleic acid molecule; an aptamer that specifically binds to a conformationally-distinct site on the target molecule; and/or an antibody, antigen binding fragment thereof, or antigen binding peptide that selectively binds to the control marker in the sample. Nucleic acid and amino acid sequences for many cell markers are known in the art and can be used to produce such reagents for detection.

The assay systems and methods of the present invention can be used not only to identify patients that are predicted to be responsive to tyrosine kinase inhibitor chemotherapeutic agents, but also to identify treatments that can improve the responsiveness of cancer cells which are resistant to tyrosine kinase inhibitor chemotherapeutic agents, and to develop adjuvant treatments that enhance the response of cancer patients to tyrosine kinase inhibitor chemotherapeutic agent(s).

The Examples that follow are illustrative of specific embodiments of the invention, and various uses thereof. They are set forth for explanatory purposes only, and are not to be taken as limiting the invention.

### EXAMPLES

Example 1: This example illustrates the RT-PCR assay performed for detecting the presence of *NTRK1-MPRIP* fusion gene.

After RNA extraction of a formalin-fixed, paraffin-embedded (FFPE) tumor section sample, a gene specific RT-PCR method was used to identify the *NTRK1-MPRIP* fusion gene. The resulting RT-PCR reaction generated an approximately 280bp fragment (see Figure 3B) that upon sequencing confirmed the presence of a novel in-frame *NTRK1-MPRIP* fusion gene in which exon 21 of *MPRIP* is fused to exon 14 of *NTRK1.* The primer sequences for RT PCR and cloning are as follows:
Primer Name : Primer Sequence (5' to 3')
MPRIPStart: ACCATGTCGGCAGCCAAGGAGAACCCGTGC (SEQ ID NO:2)
MPRIP CC1F1: ACACACGAGCTGACCTCTCTGC (SEQ ID NO:3)
MPRIP CC2F1: GTGCCTGGAGAATGCCCATCTG (SEQ ID NO:4)
MPRIP CC3F1: GCGAAGGCTAAGGCTGACTGTG (SEQ ID NO:5)
MPRIP XhoR1: CCATTGCTGCAAACCCTCGCTC (SEQ ID NO:6)
EcoRI MPRIP - Kozak ATG:GAATTCGCCGCCGCGCCGACCATGTCGG (SEQ ID NO:7)
NTRK1Y490R1: CGGCGCTTGATGTGGTGAAC (SEQ ID NO:8)
NTRK1stopR1: TATTCCGGCTAACCACTCCCAG (SEQ ID NO:9)
NTRK1stopR2: CCTAGCCCAGGACATCCAGG (SEQ ID NO:10)
NTRK1 HAstop Not1:

RNA extraction from FFPE and Frozen tissues: RNA from FFPE was processed using the RECOVERALL™ Total Nucleic Acid Isolation Kit [Ambion (Austin, Tx)]. Sections were initially deparaffinized in xylene and washed with 100% ethanol prior to the Protease K digest. After Protease K digest samples were processed for RNA isolation per manufacture instructions.

*NTRK1-MPRIP* RT-PCR: To identify the fusion breakpoint of *MPRIP* to *NTRK1* from the RNA sample, RT-PCR was carried out using the SUPERSCRIPT™ III First-Strand Synthesis System (Invitrogen) with a *NTRK1* primer located in exon 15 of *NTRK1* (NTRK Y490R1). For first strand synthesis, RNA, dNTPs and *NTRK1* Y490F1 primer were initially denatured at 65°C for 5 mins and then placed on ice for 2 mins. SUPERSCRIPT™ III reserve transcriptase, RNasin, DTT and reaction buffer was then added to the denatured samples and first strand synthesis was carried out in a PCR machine under the following conditions: 55°C, 10mins; 50°C 120mins; 70°C, 15mins; 4°C hold. Following first strand synthesis, the duplexed RNA was removed by an RNase H digest at 37°C for 20mins. RT-PCR was then performed to amplify *NTRK1-MPRIP* fusion using the same *NTRK1* reverse primer, *NTRK1* Y490R1 and a primer to *MPRIP* located in its 3^{rd} coil-coiled domain (*MPRIP* CC3F1). PCR conditions for detecting the *NTRK1* fusion: initial denaturation at 95°C for 5mins; 40 cycles of PCR (95°C for 30 sec, annealing at 58°C for 30 sec, and 30 sec extension at 72°C). PCR products were resolved on a 1.5% agarose gel and the fragments were treated with EXOSAPIT™ (Affymetrix) to remove reaction primers and unincorporated dNTPs. The RT-PCR products were sequenced by the University of Colorado Cancer Center DNA Sequencing and Analysis Core using the same forward and reverse primer in the RT-PCR reaction. The reference sequences used for exon alignment are NCBI Reference Sequences: NM_002529.3 (*NTRK1*) and NM_015134.3 (*MPRIP*).

Example 2: This example illustrates the design of FISH gene fusion probe sets to detect chromosomal rearrangements involving the *NTRK1* and *MPRIP* genes generated by the t(1;17) (q23.1;p11.2) translocation and the break apart probe sets to detect chromosomal rearrangements involving the 5' and 3' ends of the *NTRK1* gene mapping at 1q23.1. The FISH *NTRK1-MPRIP* fusion probes and *NTRK1* break apart probe sets were developed and validated as described below.

**Table 1. Detailed features of the reagents used for the NTRK1 beak-apart probe set and the MPRIP/NTRK1 fusion probe set.**

| Probe | Chromosome Band | BAC clones | Start Point | End point | Length (bp) | Primers | | SEQ ID NO: | Probe length (Kb) | Labeled color |
|---|---|---|---|---|---|---|---|---|---|---|
| 5' NTRK1 | 1q23.1 | RP11-891L18 | 156,512,039 | 156,693,389 | 181,351 | Forward | TTCCCAGCTTCTAAGATTCCACCT | 12 | 339.4 | Green |
| | | | | | | Reverse | TTTCCCGTGACATTTGGTCCCTTT | 13 | | |
| | 1q23.1 | RP11-711O18 | 156,664,877 | 156,851,480 | 186,604 | Forward | TGCATCGAAGTTTGGTTACGGGTT | 14 | | |
| | | | | | | Reverse | ACTGGAAATGCTTTGAGGTCAGGA | 15 | | |
| 3' NTRK1 | 1q23.1 | RP11-1038N13 | 156,854,507 | 156,983,651 | 129,145 | Forward | TGAAAGCCTTCATAGGTGCCTCTT | 16 | 331.8 | Red |
| | | | | | | Reverse | TGCAATCAGGGCTGTGAAAGATGT | 17 | | |
| | 1q23.1 | RP11-1059C21 | 156,972,753 | 157,186,313 | 213,561 | Forward | AAACCCAGCCACGAATCTCTTCAA | 18 | | |
| | | | | | | Reverse | ACTTGGAAGGAGTGCTGTTGTGTA | 19 | | |
| 5' MPRIP | 17p11.2 | RP11-125I16 | 16,753,286 | 16,915,525 | 162,240 | Forward | TCTAGTGCAAGGCTCTTCCTCACA | 20 | 341.4 | Green |
| | | | | | | Reverse | AGACAGCGGAGTGGAGAAGTTGAA | 21 | | |
| | 17p11.2 | RP11-796J19 | 16,916,283 | 17,094,721 | 178,439 | Forward | AAGCATGACCTCCAGGGATCTTCA | 22 | | |
| | | | | | | Reverse | ATGTGCTTCTGTCCGTGTTCCCTA | 23 | | |

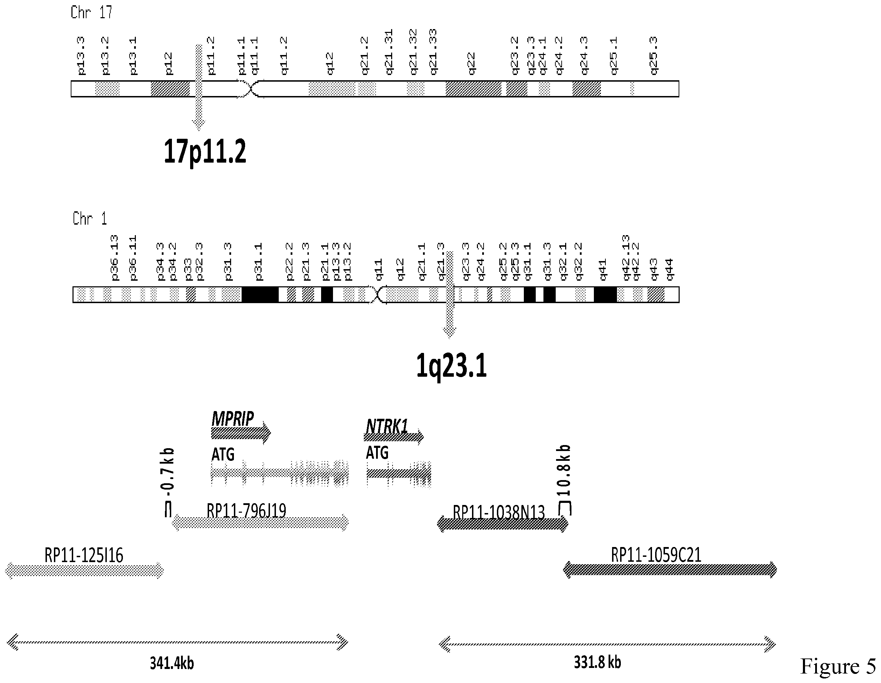

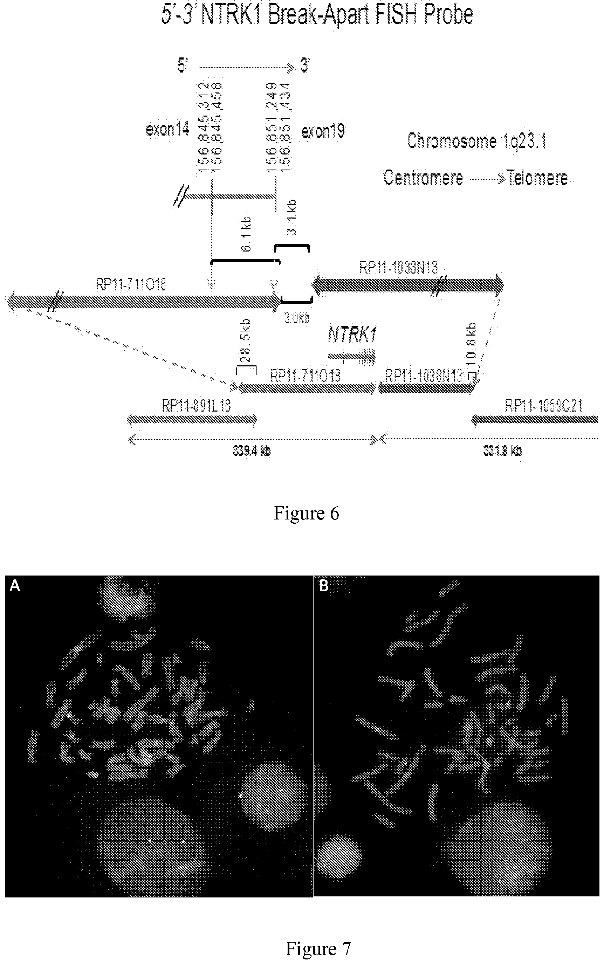

Probe Development: Clone selection, PCR verification, DNA extraction, amplification, and labelling: For the *NTRK1-MPRIP* fusion probe set, four BAC clones were selected: two for the *3' NTRK1* probe (labeled with SpectrumRed, SR), recognizing sequences at and downstream (3') the exon 20 of the *NTRK1* gene (RP11-1038N13 and RP11-1059C21), and two for the 5' *MPRIP* probe (labeled with SpectrumGreen, SG), recognizing sequences at and upstream (5') of the exon 29 of the *MPRIP* gene (RP11-796J19, and RP11-125116). For the Break Apart *NTRK1* probe set, two additional BACs (RP11-711018 and RP11-891L18) were selected mapped at and downstream of the 5' end of the *NTRK1* breakpoint. Detailed information for all 6 clones is listed in Table 1 and schematic representation of the fusion and the break-apart probe sets are shown in Figures 5 and 6 respectively.

All BAC clones were purchased from BACPAC Resources (CHORI, Oakland CA). To verify that the BAC clones encompassed the regions of interest, the specific primers were designed and synthesized by Integrated DNA Technologies. The glycerol stabs were plated on agar plates containing selected antibiotic and 10 single-cell colonies from each BAC clone were selected for PCR verification. Two PCR-validated single colonies per BAC clone had aliquots frozen in glycerol stocks at -86°C.

Mini-cultures of 1 or 2 validated single-cell colonies from each BAC clone were grown in antibiotic-containing LB medium, and genomic DNA was extracted and purified using QIAAMP™ DNA Mini Kit from Qiagen. The purified genomic DNAs from each BAC clone were subject to whole genomic amplification using the REPLI-g Midi Kit from Qiagen.

Amplified human DNA from each BAC clone was labeled in 1 µg aliquots with SpectrumRed conjugated dUTPs (all 3' *NTRK1* probe clones), and SpectrumGreen conjugated dUTPs (all 5' *MPRIP* probe clones and all 5' *NTRK1* probe clones), using the Vysis Nick translation kit (Cat# 32-801300), according to the manufacturer's instructions. Each reaction was then treated according to the planned use in validation assays for single clones or combos. Labeled DNAs were co-precipitated with herring sperm DNA as carrier (1:50) and human Cot-1 DNA (1:10) for blocking repetitive sequences and each pellet was diluted in 10 µl of TDENHYB™-2 hybridization buffer from Insitus Biotechnologies for a final concentration of 100 ng/µl.

Definition of Scoring System: Expected signal patterns: In the 5' *MPRIP- 3' NTRK1* FISH fusion probe, the 3' *NTRK1* probe covered 331.8Kb of the 3'genomic region from the break points of *NTRK1* gene in chromosome 1q23.1. The 5' *MPRIP* probe covered 341.4 Kb of the 5'genomic region from the break points of *MPRIP* gene in chromosome 17. Normal diploid (2N) cells should have two copies of single Red (R), and two copies of single Green (G) signals. In cells carrying a translocation t(1;17)(q23.1;p11.2), the 5' *MPRIP* sequence recognized by the (G) probe will be molecularly fused to 3' *NTRK1* sequence recognized by the (R) probe, generating a fused R/G signal. In a normal cell, due to eventual physical co-localization, one copy of R signal and one copy of G signal could be juxtaposed to each other generating a fused R/G signal that mimics the positive signal for the t(1;17). In the *5' NTRK1- 3' NTRK1* FISH break-apart probe, the 5' probe covered 339.4 kb upstream the breakpoint and the 3' *NTRK1* probe covered 331.8Kb downstream the break point of *NTRK1* in chromosome 1q23.1. Normal diploid (2N) cells should have two copies of fused R/G signals and cells carrying a translocation t(1;17)(q23.1;p11.2) should display at least one copy of single G and single R signals.

Analyses of Signal Configuration on Normal Specimens: Typical fluorescent signals are seen as a round and compact spot, named "dot" (see Figure 30). Atypical signal patterns may be seen in nuclei due to technical variations, probe quality and chromatin stretching. Because the fusion *5' MPRIP- 3' NTRK1* and the break-apart *5' NTRK1- 3' NTRK1* probe sets are designed for differentiation between normal and rearranged genes in tumor cells, any atypical configuration of the signals or the relationship (signal pattern) between both probes in a set should be evaluated in detail in normal specimens.

The configuration "Dot" is the typical round and compact signal, as described above (and see Figure 30). The other categories of split, patchy, and stringy are uncompact signals: split is a divided signal, usually in 2 or 3 fragments; patchy is a diffuse signal with irregular presentation and multiple tiny spots; stringy is an elongated fibrous-like signal. All four of these signal configurations were presented in cell suspension assays. For signal scoring, one dot was counted as 1 signal; a signal with split, patchy or stringy configuration was also counted as 1, even if it consists of 2 or more small spots.

Example 3: This example illustrates that the FISH fusion probe set for the detection of the *MPRIP*/*NTRK1* gene fusion works efficiently both in cell suspensions and FFPE specimens.

Validation of DNA from single BAC clones and for each of the sets *5' MPRIP,* 5' *NTRK1* and 3' *NTRK1*: For validation of the single BAC clones for the Fusion probe set, dual-color FISH assays were performed using combinations of one 3' *NTRK1* probe and one 5' *MPRIP* probe [Clones (RP11-1038N13+ RP11-125I16) and (RP11-1059C21+ RP11-796J19)] in the cell line GM09948 (normal karyotype 46,XY). All of the probe mixtures were made up of 100ng of each of the clones involved and the total volume made up to 4.5 µl with CDENHYB™-2 hybridization buffer for a hybridization area of 113mm².

FISH assay: All FISH assays in cell suspensions were performed according to standard protocol. Briefly, the slides were treated in a solution of 70% acetic acid for 20∼30 sec, incubated in 0.008% pepsin/0.01 M HCL at 37°C for 3∼5 min, fixed in 1% formaldehyde solution for 10 min and dehydrated in graded ethanol series. Probe mix was applied to the selected 12 mm² diameter hybridization areas, which were covered with glass cover slips and sealed with rubber cement. DNA co-denaturation was performed for 8 minutes in an 85°C dry oven and hybridization was allowed to occur in a moist chamber at 37°C for 40 hours. Post-hybridization washes were performed with 2×SSC/0.3% NP-40 at 72°C for 2 min, and 2×SSC for 2 min at room temperature, and dehydrated in graded ethanol series. Chromatin was counterstained with DAPI (03µg/ml in Vectashield Mounting Medium, Vector Laboratories).

FISH assays in FFPE specimens were also performed according to standard lab protocols. Specimens were incubated at 56°C for 4 h, dewaxed in CitriSolv, dehydrated and air-dried, then slides were soaked in 2xSSC at 75°C for 13∼14 min and digested in 0.6 mg/ml proteinase K at c for 14∼16 min. After dehydration, the fusion probe set *5' MPRIP- 3' NTRK1* which contained 100ng of each of the 4 BAC clones in 4.5 µl of hybridization buffer was applied to selected 113 mm² hybridization areas and hybridization was allowed to occur for ∼40 h in a humidified chamber at 37°C. Post-hybridization washes were performed as described above for the cell line.

### Evaluation of Single BAC Clone probes in Non-Rearranged Cell Lines

Chromosome Mapping: The quality of the preparations and the intensity of the fluorescence signal were excellent in all slides. Chromosomal mapping was investigated in 25 karyotypically normal metaphase spreads and all of the individual BAC clones mapped correctly: the BAC clones RP11-1038N13 and RP11-1059C21 for 3' *NTRK1* mapped at 1q23.1, the BAC clones RP11-796J19, and RP11-125I16 for 5' *MPRIP* mapped at 17p11.2 and the BAC clones RP11-711O18/ RP11-891L18 for 5' *NTRK1* also mapped at 1q23.1. The GM09948 cell line had about 99% of the cells with diploid (2N) and 1% of the cells with tetraploid (4N) chromosome content. These cells had, respectively, 2 and 4 copies of each of the clones tested. See Figures 7 and 8.

Analysis of Signal Configuration: The signal quality and pattern classification were investigated in 100 diploid interphase nuclei (2N) and the results are summarized in the Table 2.

**Table 2. Distribution of signals of the MPRIP/NTRK1 probe set in disomic interphase cells according to the configuration.**

| **Signal Configuration** | **Frequency** | **3'NTRK1 (Red)** | **5' MPRIP (G)** |
|---|---|---|---|
| **Dot** | N | 118 | 99 |
| | % | 58.7 | 47.8 |
| **Split** | N | 61 | 52 |
| | % | 30.4 | 25.1 |
| **Patchy** | N | 12 | 37 |
| | % | 6.0 | 17.9 |
| **Stringy** | N | 10 | 19 |
| | % | 5.0 | 9.2 |
| **Total** | N | 201 | 207 |
| | % | 100 | 100 |

| | | | |
|---|---|---|---|
| Evaluation in FFPE lung cancer sections | | | |

Three non-small cell lung cancer specimens were tested with the *NTRK1-MPRIP* fusion probe set and results of the analyses are shown in Table 3.

Two specimens were negative for the presence of fusion, with 8% and 9% of cells displaying a typical pattern for positivity. Specimen S-12-047486 (Figure 8A) had low copy number of each DNA target while specimen S-12-047098 (Figure 8B) had much higher copy number for both tested targets, albeit higher for *NTRK1* than *MPRIP.* Conversely, the third specimen S-12-6988 B1 showed 88% of cells with typical pattern for *NTRK1-MPRIP* gene fusion, a clear support for a positive result. Interestingly, as illustrated in Figure 10, this specimen was heterogeneous, with tumor nuclei ranging in size from medium to very large and harboring, respectively, few copies of each DNA target with one copy of the *NTRK1-MPRIP* fusion to gene amplification of the *NTRK1-MPRIP* fusion. These two extreme patterns are illustrated in Figures 9A and 9B, respectively.

Example 4: This example illustrates that the FISH break apart probe set for the detection of the *5'NTRK1*/*3'NTRK1* gene rearrangement works efficiently both in cell suspensions and FFPE specimens.

The *5'NTRK1*/*3'NTRK1* Break Apart probe set was validated in the cell line GM09948 (normal karyotype 46,XY) by using the two 3' *NTRK1* probes and the two 5' *NTRK1* probes, (Clones RP11-1038N13/RP11-1059C21+ RP11-711O18/ RP11-891L18), because using only one clone of each set will generate split signals, using the same methodology as described above for gene fusion probes. Figures 10a-10d show the results. *5'NTRK1* (green signal) is the proximal end of the gene with respect to the centromere and *3'NTRK1* (red signal) is the distal end of the gene with respect to the centromere. Both probes mapped correctly at 1q23.1.

The *5'NTRK1*/*3'NTRK1* Break Apart probe set was tested in the GM09948 cell line and specimen S12-6988 B1 as described in Example 3. Analysis was performed on epifluorescence microscope using single interference filters sets for green (FITC), red (Texas red) and blue (DAPI). For each interference filter, monochromatic images were acquired and merged using CytoVision (Leica Microsystems Inc).

The quality of the preparations and the intensity of the fluorescence signals were adequate in all the slides. Chromosomal mapping was investigated in metaphase spreads and the individual BAC clones mapped correctly at 1q23.1 (Figures 10a-10d). Evaluation in FFPE lung cancer section was done with specimen S12-6988 B1 (which was shown in Example 3 to have the 5' *MPRIP- 3' NTRK1* rearrangement) and positive patterns were observed (Figure 11). As shown in Figure 11 cells show both the 'positive' pattern of split red and green and the 'normal' pattern of Fused red and green signals.

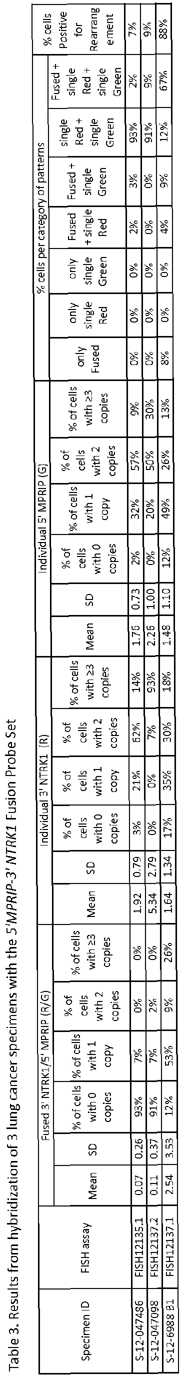

Example 5: This example demonstrates that activated TRKA (the protein product of the NTRK1 gene) can be inhibited by several small molecule tyrosine kinase inhibitors.

Gene fusion events lead to activation of the kinase domain encoded in the 3' end of the fusion gene by leading to increased expression via the promoter of the 5' gene in the fusion and often by inducing dimerization through domains (such as coiled-coil domains) encoded within the 5' gene fusion partner. This enhanced dimerization leads to constitutive activation of the kinase domain, in this case the kinase domain of TRKA.

To determine whether small molecular tyrosine kinase inhibitors inhibit TRKA, the inventors expressed full length TRKA in 293T cells (Figures 12 and 13). The NTRK1 gene was cloned into pCDH-MCS1-EF1-puro with the addition of a hemagglutinin (HA) tag at the 3' (C-terminal) end. This vector was transiently transfected into 293T cells which demonstrated expression of TRKA compared to the empty vector control using an antibody to both HA (Cell Signaling, C29F4) and TRKA (Santa Cruz, C-118) in immunoblot analysis (Figure 12A). Figure 12A shows expression of an approximate 115-120kD protein detected by an HA-specific antibody (left, Cell Signaling) and a TRKA-specific antibody (right, Santa Cruz, SC-118). Immunoprecipation of TRKA using the HA-specific antibody followed by immunobloting with an anti-phosphotyrosine antibody (Millipore, 4G10) demonstrated significant phosphorylation of TRKA in the DMSO-treated (control) sample (Figure 12B). Phosphorylation of TRKA was significantly reduced after treatment of cells with 1 µM of either K252A, crizotinib, or CEP-701 (all purchased from Selleck Chemical) for 5 hours, indicating inhibition of TRKA activity. Figure 12B shows immunoprecipation using an HA-specific antibody (Cell Signaling) followed by immunoblot using the same antibody (left) or a phosphotyrosine specific antibody (right) following treatment with 1 µM of the indicated inhibitors or DMSO (control) for 5 hours. Figure 12C shows that expression of NTRK1-MPRIP yields a chimeric protein that is autophosphorylated. Immunoblot analysis of 293T cells transiently transfected with empty vector (EV), full length NTRK1 cDNA, NTRK1-MPRIP cDNA compared to tumor cells from a frozen pleural fluid sample or early passage cells in culture (CUTO-3) from the index patient with the NTRK1-MPRIP fusion gene.

Expression of TRKA was detected in cell lysates and this protein was activated in the absence or presence of its ligand, nerve growth factor (NGF), as detected by immunoblot analysis using antibodies to phosphotyrosines at TRKA amino acid positions 490, 674 and 675 (Cell Signaling) (Figure 13A). Further evidence of TRKA activity was seen by increased phosphorylation of downstream signaling pathways such as AKT (Cell Signaling, S473) or ERK (Cell Signaling). Treatment of cells with 1 µM of either K252A or crizotinib led to decreased phosphorylation of TRKA and ERK, whereas CEP-701 also inhibited phosphorylation of AKT in addition to TRKA and ERK. Figure 13 shows SDS-PAGE of 293T cell lysates with expression of TRKA-HA or empty vector in the presence or absence of NGF (10 minutes) and the presence or absence of the indicated tyrosine kinase inhibitors at 1 µM for 5 hours. Membranes were probed with antibodies to TRKA phosphotyrosine 490, 674, and 675 (Cell Signaling), total TRKA (anti-HA, Cell Signaling), AKT phosphoserine 473 (Cell Signaling), total AKT (Cell Signaling), phosphorylated ERK p42/44 (Cell Signaling), total ERK p42/44 (Cell Signaling), and tubulin (Santa Cruz, SC-8035).

Those skilled in the art will appreciate, or be able to ascertain using no more than routine experimentation, further features and advantages of the invention based on the above-described embodiments. Accordingly, the invention is not to be limited by what has been particularly shown and described, except as indicated by the appended Claims.
1. Ferlay J, Shin HR, Bray F, Forman D, Mathers C, Parkin DM. Estimates of worldwide burden of cancer in 2008: GLOBOCAN 2008. Int J Cancer 2010;127:2893-917.
2. Wells SA, Jr., Gosnell JE, Gagel RF, et al. Vandetanib for the treatment of patients with locally advanced or metastatic hereditary medullary thyroid cancer. J Clin Oncol 2010;28:767-72.
3. Mok TS, Wu YL, Thongprasert S, et al. Gefitinib or carboplatin-paclitaxel in pulmonary adenocarcinoma. N Engl J Med 2009;361:947-57.
4. Kwak EL, Bang YJ, Camidge DR, et al. Anaplastic lymphoma kinase inhibition in non-small-cell lung cancer. N Engl J Med 2010;363:1693-703.
5. Soda M, Choi YL, Enomoto M, et al. Identification of the transforming EML4-ALK fusion gene in non-small-cell lung cancer. Nature 2007;448:561-6.
6. Rikova K, Guo A, Zeng Q, et al. Global survey of phosphotyrosine signaling identifies oncogenic kinases in lung cancer. Cell 2007;131:1190-203.
7. Takeuchi K, Choi YL, Togashi Y, et al. KIF5B-ALK, a novel fusion oncokinase identified by an immunohistochemistry-based diagnostic system for ALK-positive lung cancer. Clin Cancer Res 2009;15:3143-9.
8. Birchmeier C, Sharma S, Wigler M. Expression and rearrangement of the ROS1 gene in human glioblastoma cells. Proc Natl Acad Sci U S A 1987;84:9270-4.
9. Charest A, Lane K, McMahon K, et al. Fusion of FIG to the receptor tyrosine kinase ROS in a glioblastoma with an interstitial del(6)(q21q21). Genes Chromosomes Cancer 2003;37:58-71.
10. Bergethon K, Shaw AT, Ou SH, et al. ROS1 rearrangements define a unique molecular class of lung cancers. J Clin Oncol 2012;30:863-70.
11. Rimkunas VM, Crosby K, Kelly M, et al. Analysis of Receptor Tyrosine Kinase ROS1 Positive Tumors in Non-small Cell Lung Cancer: Identification of a FIG-ROS1 Fusion. Clin Cancer Res 2012.
12. Takeuchi K, Soda M, Togashi Y, et al. RET, ROS1 and ALK fusions in lung cancer. Nat Med 2012;18:378-81.
13. Davies KD, Le AT, Theodoro MF, et al. Identifying and Targeting ROS1 Gene Fusions in Non-Small Cell Lung Cancer. Clin Can Res;In press.
14. Nikiforov YE, Nikiforova MN. Molecular genetics and diagnosis of thyroid cancer. Nat Rev Endocrinol 2011;7:569-80.
15. Herbst RS, Heymach JV, O'Reilly MS, Onn A, Ryan AJ. Vandetanib (ZD6474): an orally available receptor tyrosine kinase inhibitor that selectively targets pathways critical for tumor growth and angiogenesis. Expert Opin Investig Drugs 2007;16:239-49.
16. Doebele RC, Pilling AB, Aisner DL, et al. Mechanisms of resistance to crizotinib in patients with ALK gene rearranged non-small cell lung cancer. Clin Cancer Res 2012;18:1472-82.
17. Davies KD, Le AT, Theodoro MF, et al. Targeting ROS1 Gene Fusions in Non-Small Cell Lung Cancer. Clin Can Res Submitted.
18. Camidge DR, Kono SA, Flacco A, et al. Optimizing the detection of lung cancer patients harboring anaplastic lymphoma kinase (ALK) gene rearrangements potentially suitable for ALK inhibitor treatment. Clin Cancer Res 2010;16:5581-90.
19. Camidge DR, Theodoro M, Maxson DA, et al. Correlations between the percentage of tumor cells showing an ALK (anaplastic lymphoma kinase) gene rearrangement, ALK signal copy number, and response to crizotinib therapy in ALK fluorescence in situ hybridization-positive nonsmall cell lung cancer. Cancer 2012.
20. Pierotti MA, Bongarzone I, Borello MG, Greco A, Pilotti S, Sozzi G. Cytogenetics and molecular genetics of carcinomas arising from thyroid epithelial follicular cells. Genes Chromosomes Cancer 1996;16:1-14.
21. Hondermarck H. Neurotrophins and their receptors in breast cancer. Cytokine Growth Factor Rev 2012.
22. Knezevich SR, McFadden DE, Tao W, Lim JF, Sorensen PH. A novel ETV6-NTRK3 gene fusion in congenital fibrosarcoma. Nat Genet 1998;18:184-7.
23. Tognon C, Knezevich SR, Huntsman D, et al. Expression of the ETV6-NTRK3 gene fusion as a primary event in human secretory breast carcinoma. Cancer Cell 2002;2:367-76.
24. Kralik JM, Kranewitter W, Boesmueller H, et al. Characterization of a newly identified ETV6-NTRK3 fusion transcript in acute myeloid leukemia. Diagn Pathol 2011;6:19.
25. Cui JJ, Tran-Dube M, Shen H, et al. Structure based drug design of crizotinib (PF-02341066), a potent and selective dual inhibitor of mesenchymal-epithelial transition factor (c-MET) kinase and anaplastic lymphoma kinase (ALK). J Med Chem 2011;54:6342-63.
26. O'Hare T, Shakespeare WC, Zhu X, et al. AP24534, a pan-BCR-ABL inhibitor for chronic myeloid leukemia, potently inhibits the T3151 mutant and overcomes mutation-based resistance. Cancer Cell 2009;16:401-12.
27. Karaman MW, Herrgard S, Treiber DK, et al. A quantitative analysis of kinase inhibitor selectivity. Nat Biotechnol 2008;26:127-32.
28. George DJ, Dionne CA, Jani J, et al. Sustained in vivo regression of Dunning H rat prostate cancers treated with combinations of androgen ablation and Trk tyrosine kinase inhibitors, CEP-751 (KT-6587) or CEP-701 (KT-5555). Cancer Res 1999;59:2395-401.
29. Wang T, Yu D, Lamb ML. Trk kinase inhibitors as new treatments for cancer and pain. Expert Opin Ther Pat 2009;19:305-19.

## Claims

1. A method to select a lung cancer patient who is predicted to respond to the administration of a chemotherapeutic regimen comprising:
(a) detecting in a sample of lung tumor cells from the patient the presence or absence of a NTRK1-MPRIP gene fusion.
(b) selecting the patient as predicted to respond to the administration of a chemotherapeutic regimen comprising an agent selected from the group consisting of a tyrosine kinase inhibitor, an HSP90 inhibitor, an inhibitor of tyrosine kinase downstream signalling cascade, and combinations thereof if a NTRK1-MPRIP gene fusion is detected in the sample of tumor cells; or
(c) selecting the patient as predicted to not respond to the administration of a chemotherapeutic regimen comprising an agent selected from the group consisting of a tyrosine kinase inhibitor, an HSP90 inhibitor, an inhibitor of tyrosine kinase downstream signaling cascade, and combinations thereof if a NTRK1-MPRIP gene fusion is not detected in the sample of tumor cells.

2. The method of claim 1, wherein the detection comprises detecting a level of the NTRK1-MPRIP gene fusion present in the sample of tumor cells and, comparing the level to a standard level or reference range.

3. The method of claim 1, wherein the presence of the NTRK1-MPRIP gene fusion is determined by detecting the presence of a polynucleotide.

4. The method of claim 3, wherein the presence of the NTRK1-MPRIP gene fusion is determined by Fluorescent In Situ Hybridization (FISH).

5. The method of claim 1, wherein the presence of the NTRK1-MPRIP gene fusion is determined by detecting the presence of a polypeptide.

6. The method of claim 5, wherein the method comprises detecting the presence of the polypeptide using at least one of an antibody, an antibody derivative, and an antibody fragment, that specifically binds to the polypeptide or a fragment thereof.

7. The method of claim 1, wherein the detecting of the NTRK1-MPRIP gene fusion comprises:
obtaining RNA from the sample of tumor cells;
generating cDNA from the RNA;
amplifying the cDNA with PCR primers specific for the NTRK1-MPRIP gene fusion selected from the group consisting of
ACCATGTCGGCAGCCAAGGAGAACCCGTGC (SEQ ID NO:2);
ACACACGAGCTGACCTCTCTGC (SEQ ID NO:3);
TGCCTGGAGAATGCCCATCTG (SEQ ID NO:4);
GCGAAGGCTAAGGCTGACTGTG (SEQ ID NO:5);
CCATTGCTGCAAACCCTCGCTC (SEQ ID NO:6);
GAATTCGCCGCCGCGCCGACCATGTCGG (SEQ ID NO:7);
CGGCGCTTGATGTGGTGAAC (SEQ ID NO:8);
TATTCCGGCTAACCACTCCCAG (SEQ ID NO:9);
CCTAGCCCAGGACATCCAGG (SEQ ID NO:10); and,
determining the presence or absence of the NTRK1-MPRIP gene fusion in the amplified cDNA.

8. The method of claim 1, wherein the patient is a human.

9. The method of claim 1, wherein the tyrosine kinase inhibitor is selected from the group consisting of crizotinib, ponatinib, dovitinib, rebastinib, lestaurtinib, AZD-7451, larotrectinib, ARRY-523, ARRY-772 and combinations thereof or wherein the tyrosine kinase inhibitor is a TrkA inhibitor.

10. Use of a kit in the method of claim 1, wherein the kit comprises one or more of the following:
(a) an antibody, wherein the antibody specifically binds with a polypeptide marker,
(b) a labelled binding partner to the antibody,
(c) a solid phase upon which is immobilized the antibody or its binding partner,
(d) a polynucleotide probe that can hybridize to a polynucleotide marker,
(e) pairs of primers that under appropriate reaction conditions can prime amplification of at least a portion of a gene fusion polynucleotide marker (e.g., by PCR),
(f) instructions on how to use the kit, and
(g) a label or insert indicating regulatory approval for diagnostic or therapeutic use.

11. A tyrosine kinase inhibitor drug for use in the treatment of a lung cancer patient who is predicted to respond to the administration of a chemotherapeutic regimen in accordance with the method of claim 1.

## Patentansprüche

1. Verfahren, um einen Lungenkrebspatienten auszuwählen, für den vorhergesagt wird, dass er auf die Verabreichung einer chemotherapeutischen Behandlung anspricht, umfassend:
(a) Nachweisen der Anwesenheit oder Abwesenheit einer NTRK1-MPRIP-Genfusion in einer Probe von Lugentumorzellen des Patienten;
(b) Auswahl des Patienten als vorhergesagt, auf die Verabreichung einer chemotherapeutischen Behandlung umfassend einen Wirkstoff ausgewählt aus der Gruppe bestehend aus einem Tyrosinkinase-Inhibitor, einem HSP90-Inhibitor, einem Inhibitor der der Tyrosinkinase nachgeschalteten Signalkaskade und Kombinationen daraus, anzusprechen, wenn eine NTRK1-MPRIP-Genfusion in der Probe der Tumorzellen nachgewiesen wird; oder
(c) Auswahl des Patienten als vorhergesagt, auf die Verabreichung einer chemoterapeutischen Behandlung umfassend ein Wirkstoff ausgewählt aus der Gruppe bestehend aus einem Tyrosinkinase-Inhibitor, einem HSP90-Inhibitor, einem Inhibitor der der Tyrosinkinase nachgeschalteten Signalkaskade und Kombinationen daraus, nicht anzusprechen, wenn eine NTRK1-MPRIP-Genfusion in der Probe der Tumorzellen nicht nachgewiesen wird.

2. Verfahren nach Anspruch 1, wobei der Nachweis das Nachweisen eines Levels der NTRK1-MPRIP Genfusion, die in der Probe der Tumorzellen vorhanden ist, und das Vergleichen des Levels zu einem Standardlevel oder Referenzbereich, umfasst.

3. Verfahren nach Anspruch 1, wobei die Anwesenheit der NTRK1-MPRIP-Genfusion durch Nachweisen der Anwesenheit eines Polynukleotids bestimmt wird.

4. Verfahren nach Anspruch 3, wobei die Anwesenheit der NTRK1-MPRIP-Genfusion durch Fluoreszenz-in-situ-Hybridisierung (FISH) bestimmt wird.

5. Verfahren nach Anspruch 1, wobei die Anwesenheit der NTRK1-MPRIP-Genfusion durch Nachweisen der Anwesenheit eines Polypeptids bestimmt wird.

6. Verfahren nach Anspruch 5, wobei das Verfahren das Nachweisen der Anwesenheit des Polypeptids unter Verwendung von mindestens einem von einem Antikörper, einem Antikörperderivat und einem Antikörperfragment umfasst, der/das spezifisch an das Polypeptid oder ein Fragment davon bindet.

7. Verfahren nach Anspruch 1, wobei das Nachweisen der NTRK1-MPRIP-Genfusion umfasst:
Gewinnen von RNA aus der Probe von Tumorzellen;
Erzeugen von cDNA aus der RNA;
Amplifizieren der cDNA mit PCR Primern, die spezifisch für die NTRK1-MPRIP-Genfusion ausgewählt aus der Gruppe bestehend aus
ACCATGTCGGCAGCCAAGGAGAACCCGTGC (SEQ ID NO:2);
ACACACGAGCTGACCTCTCTGC (SEQ ID NO:3);
TGCCTGGAGAATGCCCATCTG (SEQ ID NO:4);
GCGAAGGCTAAGGCTGACTGTG (SEQ ID NO:5);
CCATTGCTGCAAACCCTCGCTC (SEQ ID NO:6);
GAATTCGCCGCCGCGCCGACCATGTCGG (SEQ ID NO:7);
CGGCGCTTGATGTGGTGAAC (SEQ ID NO:8);
TATTCCGGCTAACCACTCCCAG (SEQ ID NO:9);
CCTAGCCCAGGACATCCAGG (SEQ ID NO: 10); und, sind;
Bestimmen der Anwesenheit oder Abwesenheit der NTRK1-MPRIP-Genfusion in der amplifizierten cDNA.

8. Verfahren nach Anspruch 1, wobei der Patient ein Mensch ist.

9. Verfahren nach Anspruch 1, wobei der Tyrosinkinase-Inhibitor ausgewählt ist aus der Gruppe bestehend aus Crizotinib, Ponatinib, Dovitinib, Rebastinib, Lestaurtinib, AZD-7451, Larotrectinib, ARRY-523, ARRY-772 und Kombinationen daraus oder wobei der Tyrosinkinase-Inhibitor ein TrkA-Inhibitor ist.

10. Verwendung eines Kits in dem Verfahren nach Anspruch 1, wobei das Kit einen oder mehrere der folgenden umfasst:
(a) einen Antikörper, wobei sich der Antikörper spezifisch mit einem Polypeptidmarker bindet,
(b) einen markierten Bindungspartner des Antikörpers,
(c) eine feste Phase, auf die der Antikörper oder dessen Bindungspartner immobilisiert ist,
(d) eine Polynukleotidprobe, die an einen Polynukleotidmarker hybridisieren kann,
(e) Paare von Primern, die unter geeigneten Reaktionsbedingungen die Amplifikation von mindestens einem Teil eines Genfusions-Polynukleotidmarkers hervorrufen können (zum Beispiel durch PCR),
(f) Anleitungen zur Benutzung des Kits, und
(g) eine Kennzeichnung oder ein Beiblatt, die/das die behördliche Zulassung zur diagnostischen oder therapeutischen Verwendung anzeigt.

11. Ein Tyrosinkinase-Inhibitor-Arzneimittel zur Verwendung in der Behandlung eines Lungenkrebspatienten, für den gemäß dem Verfahren nach Anspruch 1 vorhergesagt ist, dass er auf die Verabreichung einer chemotherapeutischen Behandlung anspricht.

## Revendications

1. Procédé pour sélectionner un patient ayant un cancer du poumon dont il est prédit qu'il réagirait à l'administration d'un schéma chimiothérapeutique comprenant :
(a) la détection dans un échantillon de cellules tumorales du poumon provenant du patient de la présence ou de l'absence d'une fusion de gènes NTRK1-MPRIP,
(b) la sélection du patient comme étant prédit qu'il réagirait à l'administration d'un schéma chimiothérapeutique comprenant un agent choisi dans le groupe consistant en un inhibiteur de tyrosine kinase, un inhibiteur de HSP90, un inhibiteur de cascade de signalisation en aval de tyrosine kinase, et leurs combinaisons si une fusion de gènes NTRK1-MPRIP est détectée dans l'échantillon de cellules tumorales ; ou
(c) la sélection du patient comme étant prédit qu'il ne réagirait pas à l'administration d'un schéma chimiothérapeutique comprenant un agent choisi dans le groupe consistant en un inhibiteur de tyrosine kinase, un inhibiteur de HSP90, un inhibiteur de cascade de signalisation en aval de tyrosine kinase, et leurs combinaisons si une fusion de gènes NTRK1-MPRIP n'est pas détectée dans l'échantillon de cellules tumorales.

2. Procédé selon la revendication 1, où la détection comprend la détection d'un niveau de la fusion de gènes NTRK1-MPRIP présente dans l'échantillon de cellules tumorales et la comparaison du niveau à un niveau standard ou une plage de référence.

3. Procédé selon la revendication 1, où la présence de la fusion de gènes NTRK1-MPRIP est déterminée par détection de la présence d'un polynucléotide.

4. Procédé selon la revendication 3, où la présence de la fusion de gènes NTRK1-MPR1P est déterminée par hybridation in situ en fluorescence (FISH).

5. Procédé selon la revendication 1, où la présence de la fusion de gènes NTRK1-MPRIP est déterminée par détection de la présence d'un polypeptide.

6. Procédé selon la revendication 5, où le procédé comprend la détection de la présence du polypeptide au moyen d'au moins un d'un anticorps, d'un dérivé d'anticorps et d'un fragment d'anticorps, qui se lie spécifiquement au polypeptide ou à un fragment de celui-ci.

7. Procédé selon la revendication 1, où la détection de la fusion de gènes NTRK1-MPRIP comprend :
l'obtention d'ARN à partir de l'échantillon de cellules tumorales ;
la production d'ADNc à partir de l'ARN ;
l'amplification de l'ADNc avec des amorces de PCR spécifiques de la fusion de gènes NTRK1-MPRIP choisies dans le groupe consistant en
ACCATGTCGGCAGCCAAGGAGAACCCGTGC (SEQ ID NO:2);
ACACACGAGCTGACCTCTCTGC (SEQ ID NO:3);
TGCCTGGAGAATGCCCATCTG (SEQ ID NO:4);
GCGAAGGCTAAGGCTGACTGTG (SEQ ID NO:5);
CCATTGCTGCAAACCCTCGCTC (SEQ ID NO:6);
GAATTCGCCGCCGCGCCGACCATGTCGG (SEQ ID NO:7);
CGGCGCTTGATGTGGTGAAC (SEQ ID NO:8);
TATTCCGGCTAACCACTCCCAG (SEQ ID NO:9);
CCTAGCCCAGGACATCCAGG (SEQ ID NO:10); et
la détermination de la présence ou de l'absence de la fusion de gènes NTRK1-MPRIP dans l'ADNc amplifié.

8. Procédé selon la revendication 1, où le patient est un humain.

9. Procédé selon la revendication 1, où l'inhibiteur de tyrosine kinase est choisi dans le groupe consistant en crizotinib, ponatinib, dovitinib, rebastinib, lestaurtinib, AZD-7451, larotrectinib, ARRY-523, ARRY-772 et leurs combinaisons ou bien où l'inhibiteur de tyrosine kinase est un inhibiteur de TrkA.

10. Utilisation d'un kit dans le procédé selon la revendication 1, où le kit comprend un ou plusieurs des suivants :
(a) un anticorps, où l'anticorps se lie spécifiquement avec un marqueur polypeptidique,
(b) un partenaire de liaison de l'anticorps marqué,
(c) une phase solide sur laquelle est immobilisé l'anticorps ou son partenaire de liaison,
(d) une sonde polynucléotidique qui peut s'hybrider à un marqueur polynucléotidique,
(e) des paires d'amorces qui dans des conditions réactionnelles appropriées peuvent amorcer l'amplification d'au moins une partie d'un marqueur polynucléotidique de fusion de gènes (par exemple par PCR),
(f) des instructions sur la manière d'utiliser le kit, et
(g) une étiquette ou insert indiquant l'agrément réglementaire pour une utilisation diagnostique ou thérapeutique.

11. Médicament inhibiteur de tyrosine kinase destiné à être utilisé dans le traitement d'un patient ayant un cancer du poumon dont il est prédit qu'il réagirait à l'administration d'un schéma chimiothérapeutique selon le procédé selon la revendication 1.


### SEQUENCE LISTING

<110> THE REGENTS OF THE UNIVERSITY OF COLORADO Doebele, Robert C. Garcia, Marileila Varella Le, Anh T.
<120> METHODS FOR DIAGNOSIS AND TREATMENT OF CANCER
<130> 2848-142-PCT
<140> not yet assigned
   <141> 2013-08-30
<150> 61/696,002
   <151> 2012-08-31
<150> 61/827,514
   <151> 2013-05-24
<160> 11
<170> PatentIn version 3.5
<210> 1
   <211> 4300
   <212> DNA
   <213> Homo sapiens
<400> 1
   gcggccgcgc tgagccccta gcccgccggg agcgccaggc cggccaggcc tgcgccgccg      60
   ccgccgccgc cgtcgccgcc gcgccgacca tgtcggcagc caaggagaac ccgtgcagga     120
   aattccaggc caacatcttc aacaagagca agtgtcagaa ctgcttcaag ccccgcgagt     180
   cgcatctgct caacgacgag gacctgacgc aggcaaaacc catttatggc ggttggctgc     240
   tcctggctcc agatgggacc gactttgaca acccagtgca ccggtctcgg aaatggcagc     300
   gacggttctt catcctttac gagcacggcc tcttgcgcta cgccctggat gagatgccca     360
   cgacccttcc tcagggcacc atcaacatga accagtgcac agatgtggtg gatggggagg     420
   gccgcacggg ccagaagttc tccctgtgta ttctgacgcc tgagaaggag catttcatcc     480
   gggcggagac caaggagatc gtcagtgggt ggctggagat gctcatggtc tatccccgga     540
   ccaacaagca gaatcagaag aagaaacgga aagtggagcc ccccacacca caggagcctg     600
   ggcctgccaa ggtggctgtt accagcagca gcagcagcag cagcagcagc agcagcatcc     660
   ccagtgctga gaaagtcccc accaccaagt ccacactctg gcaggaagaa atgaggacca     720
   aggaccagcc agatggcagc agcctgagtc cagctcagag tcccagccag agccagcctc     780
   ctgctgccag ctccctgcgg gaacctgggc tagagagcaa agaagaggag agcgccatga     840
   gtagcgaccg catggactgt ggccgcaaag tccgggtgga gagcggctac ttctctctgg     900
   agaagaccaa acaggacttg aaggctgaag aacagcagct gcccccgccg ctctcccctc     960
   ccagccccag cacccccaac cacaggaggt cccaggtgat tgaaaagttt gaggccttgg    1020
   acattgagaa ggcagagcac atggagacca atgcagtggg gccctcacca tccagcgaca 1080
   cacgccaggg ccgcagcgag aagagggcgt tccctaggaa gcgggacttc accaatgaag 1140
   cccccccagc tcctctccca gacgcctcgg cttcccccct gtctccacac cgaagagcca 1200
   agtcactgga caggaggtcc acggagccct ccgtgacgcc cgacctgctg aatttcaaga 1260
   aaggctggct gactaagcag tatgaggacg gccagtggaa gaaacactgg tttgtcctcg 1320
   ccgatcaaag cctgagatac tacagggatt cagtggctga ggaggcagcc gacttggatg 1380
   gagaaattga cttgtccgca tgttacgatg tcacagagta tccagttcag agaaactatg 1440
   gcttccagat acatacaaag gagggcgagt ttaccctgtc ggccatgaca tctgggattc 1500
   ggcggaactg gatccagacc atcatgaagc acgtgcaccc gaccactgcc ccggatgtga 1560
   ccagctcgtt gccagaggaa aaaaacaaga gcagctgctc ttttgagacc tgcccgaggc 1620
   ctactgagaa gcaagaggca gagctggggg agccggaccc tgagcagaag aggagccgcg 1680
   cacgggagcg gaggcgagag ggccgctcca agacctttga ctgggctgag ttccgtccca 1740
   tccagcaggc cctggctcag gagcgggtgg gcggcgtggg gcctgctgac acccacgagc 1800
   ccctgcgccc tgaggcggag cctggggagc tggagcggga gcgtgcacgg aggcgggagg 1860
   agcgccgcaa gcgcttcggg atgctcgacg ccacagacgg gccaggcact gaggatgcag 1920
   ccctgcgcat ggaggtggac cggagcccag ggctgcctat gagcgacctc aaaacgcata 1980
   acgtccacgt ggagattgag cagcggtggc atcaggtgga gaccacacct ctccgggaag 2040
   agaagcaggt gcccatcgcc cccgtccacc tgtcttctga agatgggggt gaccggctct 2100
   ccacacacga gctgacctct ctgctcgaga aggagctgga gcagagccag aaggaggcct 2160
   cagaccttct ggagcagaac cggctcctgc aggaccagct gagggtggcc ctgggccggg 2220
   agcagagcgc ccgtgagggc tacgtgctgc aggccacgtg cgagcgaggg tttgcagcaa 2280
   tggaagaaac gcaccagaag aagattgaag atctccagag gcagcaccag cgggagctag 2340
   agaaacttcg agaagagaaa gaccgcctcc tagccgagga gacagcggcc accatctcag 2400
   ccatcgaagc catgaagaac gcccaccggg aggaaatgga gcgggagctg gagaagagcc 2460
   agcggtccca gatcagcagc gtcaactcgg atgttgaggc cctgcggcgc cagtacctgg 2520
   aggagctgca gtcggtgcag cgggaactgg aggtcctctc ggagcagtac tcgcagaagt 2580
   gcctggagaa tgcccatctg gcccaggcgc tggaggccga gcggcaggcc ctgcggcagt 2640
   gccagcgtga gaaccaggag ctcaatgccc acaaccagga gctgaacaac cgcctggctg 2700
   cagagatcac acggttgcgg acgctgctga ctggggacgg cggtggggag gccactgggt 2760
   caccccttgc acagggcaag gatgcctatg aactagaggt cttattgcgg gtaaaggaat 2820
   cggaaataca gtacctgaaa caggagatta gctccctcaa ggatgagctg cagacggcac 2880
   tgcgggacaa gaagtacgca agtgacaagt acaaagacat ctacacagag ctcagcatcg     2940
   cgaaggctaa ggctgactgt gacatcagca ggttgaagga gcagctcaag gctgcaacgg     3000
   aagcactggg ggagaagtcc cctgacagtg ccacggtgtc cggatatggc ccggctgtgc     3060
   tggctccaga ggatgggctg gccatgtccc tgcatttcat gacattgggt ggcagctccc     3120
   tgtcccccac cgagggcaaa ggctctgggc tccaaggcca catcatcgag aacccacaat     3180
   acttcagtga tgcctgtgtt caccacatca agcgccggga catcgtgctc aagtgggagc     3240
   tgggggaggg cgcctttggg aaggtcttcc ttgctgagtg ccacaacctc ctgcctgagc     3300
   aggacaagat gctggtggct gtcaaggcac tgaaggaggc gtccgagagt gctcggcagg     3360
   acttccagcg tgaggctgag ctgctcacca tgctgcagca ccagcacatc gtgcgcttct     3420
   tcggcgtctg caccgagggc cgccccctgc tcatggtctt tgagtatatg cggcacgggg     3480
   acctcaaccg cttcctccga tcccatggac ctgatgccaa gctgctggct ggtggggagg     3540
   atgtggctcc aggccccctg ggtctggggc agctgctggc cgtggctagc caggtcgctg     3600
   cggggatggt gtacctggcg ggtctgcatt ttgtgcaccg ggacctggcc acacgcaact     3660
   gtctagtggg ccagggactg gtggtcaaga ttggtgattt tggcatgagc agggatatct     3720
   acagcaccga ctattaccgt gtgggaggcc gcaccatgct gcccattcgc tggatgccgc     3780
   ccgagagcat cctgtaccgt aagttcacca ccgagagcga cgtgtggagc ttcggcgtgg     3840
   tgctctggga gatcttcacc tacggcaagc agccctggta ccagctctcc aacacggagg     3900
   caatcgactg catcacgcag ggacgtgagt tggagcggcc acgtgcctgc ccaccagagg     3960
   tctacgccat catgcggggc tgctggcagc gggagcccca gcaacgccac agcatcaagg     4020
   atgtgcacgc ccggctgcaa gccctggccc aggcacctcc tgtctacctg gatgtcctgg     4080
   gctagggggc cggcccaggg gctgggagtg gttagccgga atactggggc ctgccctcag     4140
   catcccccat agctcccagc agccccaggg tgatctcaaa gtatctaatt caccctcagc     4200
   atgtgggaag ggacaggtgg gggctgggag tagaggatgt tcctgcttct ctggcaaggt     4260
   cccgtcatag caattatatt tattatccct tgaaaaaaaa                           4300
<210> 2
   <211> 30
   <212> DNA
   <213> Artificial Sequence
<220>
   <223> Synthetic primer
<400> 2
   accatgtcgg cagccaagga gaacccgtgc 30
<210> 3
   <211> 22
   <212> DNA
   <213> Artificial Sequence
<220>
   <223> Synthetic primer
<400> 3
   acacacgagc tgacctctct gc 22
<210> 4
   <211> 22
   <212> DNA
   <213> Artificial Sequence
<220>
   <223> Synthetic primer
<400> 4
   gtgcctggag aatgcccatc tg 22
<210> 5
   <211> 22
   <212> DNA
   <213> Artificial Sequence
<220>
   <223> Synthetic primer
<400> 5
   gcgaaggcta aggctgactg tg 22
<210> 6
   <211> 22
   <212> DNA
   <213> Artificial Sequence
<220>
   <223> Synthetic primer
<400> 6
   ccattgctgc aaaccctcgc tc 22
<210> 7
   <211> 28
   <212> DNA
   <213> Artificial Sequence
<220>
   <223> Synthetic primer
<400> 7
   gaattcgccg ccgcgccgac catgtcgg 28
<210> 8
   <211> 20
   <212> DNA
   <213> Artificial Sequence
<220>
   <223> Synthetic primer
<400> 8
   cggcgcttga tgtggtgaac 20
<210> 9
   <211> 22
   <212> DNA
   <213> Artificial Sequence
<220>
   <223> Synthetic primer
<400> 9
   tattccggct aaccactccc ag 22
<210> 10
   <211> 20
   <212> DNA
   <213> Artificial Sequence
<220>
   <223> Synthetic primer
<400> 10
   cctagcccag gacatccagg 20
<210> 11
   <211> 55
   <212> DNA
   <213> Artificial Sequence
<220>
   <223> Synthetic primer
<400> 11
   cgcggccgct taagcgtagt ctgggacgtc gtatgggtag cccaggacat ccagg 55